# EUROPEAN PATENT APPLICATION

(11) **EP 4 527 407 A1**
(43) Date of publication of application: **26.03.2025**
(21) Application number: 24159144.5
(22) Date of filing: 22.02.2024
(51) Int. Cl.: A61K 39/00, C07K 14/725, C07K 16/28, C07K 16/30

(54) **ANTI-MESOTHELIN ANTIBODIES AND USES THEREOF**

(30) Priority: 20.09.2023 KR 20230125813; 16.02.2024 KR 20240022654; 16.02.2024 WO PCT/KR2024/095352
(71) Applicant: UCI Therapeutics Inc., Seoul 04784 (KR)
(72) Inventor: JEONG, Su Yeong, 02532 Seoul (KR); YOON, Sang Won, 04582 Seoul (KR); SEO, Min Koo, 10595 Goyang-si (KR); AN, Jun Yop, 08335 Seoul (KR); HONG, Byoungseok, 06998 Seoul (KR)
(74) Representative: Schnappauf, Georg

(57) **Abstract**

The present invention relates to humanized antibodies or antigen-binding fragments capable of binding specifically to mesothelin antigen and various uses thereof.

## Description

### Technical Field

The present invention relates to antibodies or humanized antibodies having excellent binding capacity to mesothelin antigen and various uses thereof.

### Background Art

It is known that mesothelin (MSLN) is expressed at relatively low levels on mesothelial cells lining the pleura, peritoneum, and pericardium of healthy individuals (Hassan et al., American Journal of Clinical Pathology, Volume 124, Issue 6, December 2005, pages 838-845), but is highly expressed in various cancers, including mesotheliomas, squamous cell carcinomas, pancreatic cancer, lung cancer, gastric cancer, breast cancer, endometrial cancer, and ovarian cancer. The normal biological function of mesothelin is not known. However, it has been found that, in ovarian cancer, cholangiocarcinoma, lung adenocarcinoma, and triple-negative breast cancer, in which mesothelin is overexpressed, high expression levels of mesothelin are correlated with poor prognosis of the patients.

Based on this fact that mesothelin is highly expressed in tumor cells compared to normal cells, several antibody-based therapies targeting MSLN have been developed, and the therapeutic activities of mesothelin-specific antibodies have been tested, predominantly in mesothelioma, pancreas, and non-small cell lung cancer (Hassan et al., J Clin Oncol. 2016 Dec;34(34):4171-4179). More specifically, the strategies employed include direct tumor cell killing through the use of anti-MSLN antibodies, such as amatuximab, with antibody-dependent cellular cytotoxicity (ADCC) activity, as well as the use of antibody drug conjugates (ADCs), such as SS1P-PE38 and anetumab-ravtansine, comprising an antibody or antibody fragment conjugated to a toxin. In addition, anti-MSLN binding Fv fragments have been used in chimeric antigen receptor T cell therapies, and bispecifics, such as ABBV-428, are emerging from preclinical studies to clinical trials.

Because there is currently a large unmet clinical need for cancer therapy, continued development of drugs targeting mesothelin is necessary. Although some monoclonal antibodies against mesothelin have been developed in the prior art, most of these antibodies have limitations such as immunogenicity, low affinity, and low specificity. Therefore, there is still a need to develop new anti-mesothelin antibodies having stronger binding affinity for mesothelin, better specificity, stronger ADCC and CDC activities, and better antitumor activity.

Meanwhile, for the production of antibodies, monoclonal antibodies are mainly produced using mice. However, non-human antibodies, such as mouse-derived monoclonal antibodies, have problems in that they trigger an immune response because they are considered foreign antigens in the human body, and in that they have a short half-life, which limits their therapeutic effect. In order to overcome these problems, humanized antibodies have been developed in which antibody regions excluding antigen-binding CDRs are replaced with a human antibody. The currently used method of replacing a mouse antibody with a humanized antibody comprises selecting the human antibody gene most similar to the antibody to be replaced, and replacing only the CDRs of the mouse antibody with the CDRs of the human antibody by a method called CDR transplantation. Such humanized antibodies have the advantage of being capable of reducing immune responses in the human body because most of the genes have been humanized.

### DISCLOSURE

### Technical Problem

One object of the present invention is to provide an antibody or antigen-binding fragment capable of binding specifically to mesothelin.

Another object of the present invention is to provide a humanized antibody developed based on the above-described antibody, which is a novel humanized antibody or an antigen binding fragment thereof that is capable of binding to mesothelin antigen with high affinity, yet exhibits low immunogenicity when administered to the human body.

Still another object of the present invention is to provide a nucleic acid molecule encoding the above-described antibody or antigen-binding fragment, an expression vector containing the same, and a host cell transfected with the vector.

Yet another object of the present invention is to provide the use thereof for diagnosing or treating a disease in which mesothelin is overexpressed.

However, objects to be achieved by the present invention are not limited to the objects mentioned above, and other objects not mentioned above may be clearly understood by those skilled in the art from the following description.

### Technical Solution

### 1. Antibody or Antigen-Binding Fragment Thereof

According to one embodiment of the present invention, the present invention relates to an antibody or antigen-binding fragment thereof that binds specifically to mesothelin (MSLN).

As used herein, the term "mesothelin (MSLN)" is a 40- kDa human protein which is encoded by MSLN gene and expressed in mesothelial cells. Mesothelin is known to be overexpressed in several human tumors, including mesothelioma, ovarian cancer, pancreatic adenocarcinoma, lung adenocarcinoma, and cholangiocarcinoma. It is known that mesothelin binds to and interacts with MUC16, thereby contributing to the implantation and peritoneal spread of tumors by cell adhesion. In the present invention, the mesothelin may consist of the amino acid sequence set forth in SEQ ID NO: 1, without being limited thereto.

As used herein, the term "antibody" refers to a protein molecule serving as a ligand that specifically recognizes an antigen, including an immunoglobulin molecule that is immunologically reactive with a particular antigen, and the term includes all of polyclonal antibodies, monoclonal antibodies, whole antibodies, and antibody fragments. In addition, in the present invention, the antibodies include chimeric antibodies, human antibodies, humanized antibodies, bivalent bispecific molecules, minibodies, domain antibodies, bispecific or multispecific antibodies, immune cell-engaging bispecific or multispecific antibodies, antibody mimetics, unibodies, diabodies, triabodies, or tetrabodies. The term further includes single-chain antibodies (scAb) capable of binding to the neonatal Fc receptor (FcRn), derivatives of antibody constant regions, and artificial antibodies based on protein scaffolds. A full-length antibody has a structure with two full-length light chains (LCs) and two full-length heavy chains (HCs), wherein each light chain is linked to a heavy chain by a disulfide bond. The full-length antibodies include IgA, IgD, IgE, IgM, and IgG, and IgG subtypes include IgG1, IgG2, IgG3, and IgG4. In one embodiment, the antibodies provided by the present invention may be IgG antibodies.

As used herein, the term "monoclonal antibody" refers to antibody molecules of a single molecular composition obtained from substantially identical antibody population. Such monoclonal antibodies display a single binding specificity and affinity for a particular epitope. Typically, an immunoglobulin has heavy and light chains, wherein each heavy and light chain contains a constant region and a variable region (the regions are also known as "domains"). The light chain and heavy chain variable regions contain three hypervariable regions, referred to as complementarity-determining regions (CDRs), and four framework regions.

As used herein, the term "complementarity-determining region" or "CDR region" or "CDR" refers to a portion that is hypervariable in sequence in an antibody variable domain and that forms a structurally defined loop ("hypervariable loop") and/or contains an antigen contact residue ("antigen contact site"). The CDRs are primarily responsible for binding to an epitope. The complementarity-determining regions are interspersed between constant regions, called framework regions (FRs), which are relatively conservative. Each heavy chain variable region (VH) and light chain variable region (VL) consists of three CDRs and four FRs, which are arranged from an amino-terminus to a carboxyl-terminus in the following order: FR1, CDR1, FR2, CDR2, FR3, CDR3, and FR4. The heavy and light chain variable regions contain binding domains that interact with an antigen. The CDRs of the heavy chain variable region may be referred to as HCDR1, HCDR2, and HCDR3, the CDRs of the light chain variable region may be referred to as LCDR1, LCDR2, and LCDR3, the FRs of the heavy chain variable region may be referred to as HFR1, HFR2, HFR3, and HFR4, and the FRs of the light chain variable region may be referred to as LFR1, LFR2, LFR3, and LFR4. Here, the numbers are by sequential numbering from the N-terminus. In a given amino acid sequence of a light chain variable region or heavy chain variable region, the exact amino acid sequence boundary of each CDR may be determined using any one or a combination of a number of well-known antibody CDR assignment systems, including, for example: Chothia based on the three-dimensional structure of antibodies and the topology of the CDR loops (Chothia et al. (1989) Nature 342: 877-883, Al-Lazikani et al. "Standard conformations for the canonical structures of immunoglobulins", Journal of Molecular Biology, 273, 927-948 (1997)), Kabat based on antibody sequence variability (Kabat et al. Sequences of Proteins of Immunological Interest, 4th Ed. U.S. Department of Health and Human Services, National Institutes of Health (1987)), AbM (University of Bath), Contact (University College London), International ImMunoGeneTics database(IMGT) (On the World Wide Web at imgt.cines.fr/), and North CDR definitions based on affinity propagation clustering using a large number of crystal structures.

The antibody of the present invention may comprise a constant region, wherein the constant region may be derived from IgG, IgA, IgD, IgE, IgM, or a combination thereof, or a hybrid thereof, without being limited thereto. As used herein, the term "combination" means that a polypeptide encoding a single-chain immunoglobulin constant region of the same origin is linked to a single-chain polypeptide of a different origin to form a dimer or multimer. As an example, a dimer or multimer may be formed from two or more constant regions selected from the group consisting of constant regions of IgG, IgA, IgD, IgE, and IgM. In addition, the term "hybrid" means that sequences corresponding to immunoglobulin heavy chain constant regions of two or more different origins exist within a single-chain immunoglobulin heavy chain constant region. For example, a domain hybrid consisting of 1 to 4 domains selected from the group consisting of CH1, CH2, CH3, and CH4 of IgG, IgA, IgD, IgE, and IgM is possible.

As used herein, the term "full-length antibody" refers to a structure having two full-length light chains and two full-length heavy chains, wherein each light chain is linked to a heavy chain by a disulfide bond. The term includes IgA, IgD , IgE, IgM, and IgG. The IgG subtypes include IgG1, IgG2, IgG3, and IgG4.

As used herein, the terms "fragment", "binding fragment of a polypeptide", and "antibody fragment" refer to any fragment of the antibody of the invention, which retains the antigen-binding activity of the antibody, and these terms are used interchangeably. Exemplary antibody fragments include, but are not limited to, single chain antibodies, Fd, Fab, Fab', F(ab')2, dsFv, or scFv. Fd refers to a portion of the heavy chain which is included in the Fab fragment. As used herein, the term "Fab" generally refers to a fragment comprising a heavy chain variable domain and a light chain variable domain, and further comprising a light chain constant domain and a heavy chain first constant domain (CH1). The term "Fab'" generally refers to a fragment that is different from Fab by the addition of hydrophobic residues (including one or more cysteines from the hinge region of the antibody) to the carboxyl terminus of the heavy-chain CH1 domain. The term "F(ab')2" generally refers to a dimer of Fab', comprising a two Fab fragments linked by a disulfide bridge at the hinge region. The term "Fv" generally refers to the smallest antibody fragment that contains a complete antigen-recognition and antigen-binding site. In certain cases, the fragment may consist of a dimer in which a heavy chain variable region and a light chain variable region are tightly and non-covalently associated. The term "dsFv" generally refers to a disulfide bond-stabilized Fv fragment, in which the bond between a single light chain variable region and a single heavy chain variable region is a disulfide bond. The term "dAb fragment" generally refers to an antibody fragment consisting of a VH domain. In the present invention, the term "scFv" generally refers to a monovalent molecule formed by covalently connecting and pairing one heavy chain variable domain and one light chain variable domain of an antibody via a flexible peptide linker. Such scFv molecules may have a general structure of NH₂-VL-linker-VH-COOH or NH₂-VH-linker-VL-COOH.

As used herein, the term "chimeric antibody" refers to an antibody which is obtained by recombination of a variable region of a mouse antibody and a constant region of a human antibody, and has a greatly improved immune response compared to the mouse antibody.

In addition, as used herein, the term "humanized antibody" refers to an antibody obtained by modifying the protein sequence of an antibody from non-human species so that the protein sequence is similar to an antibody variant naturally produced in humans. For example, the humanized antibody may be produced by recombining mouse-derived CDRs with a human antibody-derived FR to obtain a humanized variable region, and recombining the humanized variable region with a constant region of a desired human antibody.

In addition, the term "human antibody" as used herein is intended to include antibodies or antibody variable domains having variable regions in which both the framework and CDR regions are derived from sequences of human origin. Furthermore, if the antibody or antibody variable domain contains a constant region, the constant region is also derived from such human sequences, e.g., human germline sequences, or mutated versions of human germline sequences.

As used herein, the term "anti-mesothelin antibody", "anti-mesothelin", "mesothelin antibody" or "antibody that binds to mesothelin" refers to an antibody that is able to bind to mesothelin with sufficient affinity so that the antibody may be used as a therapeutic agent targeting mesothelin. In one embodiment of the invention, the mesothelin antibody binds to mesothelin with high affinity *in vitro* or *in vivo.* Here, the binding may be determined by, for example, radioimmunoassay (RIA), biolayer interferometry (BLI), MSD assay, surface plasmon resonance (SPR), or flow cytometry (FACS), without being limited thereto.

The mesothelin-binding antibody or antigen-binding fragment of the present invention may comprise a heavy chain CDR1 comprising the amino acid sequence of at least one of SEQ ID NOs: 3 or 17; a heavy chain CDR2 comprising the amino acid sequence of at least one of SEQ ID NOs: 5 or 19; and a heavy chain CDR3 comprising the amino acid sequence of at least one of SEQ ID NOs: 7 or 21, shown in Table 1 below.

The mesothelin-binding antibody or antigen-binding fragment of the present invention may comprise a light chain CDR1 comprising the amino acid sequence of SEQ ID NO: 10; a light chain CDR2 comprising the amino acid sequence of SEQ ID NO: 12; and a light chain CDR3 comprising the amino acid sequence of at least one of SEQ ID NOs: 14 or 26, shown in Table 1 below.

The mesothelin-binding antibody or antigen-binding fragment of the present invention may comprise a heavy chain variable region comprising a heavy chain CDR1 comprising the amino acid sequence of at least one of SEQ ID NOs: 3 or 17; a heavy chain CDR2 comprising the amino acid sequence of at least one of SEQ ID NOs: 5 or 19; and a heavy chain CDR3 comprising the amino acid sequence of at least one of SEQ ID NOs: 7 or 21, and comprise a light chain variable region comprising a light chain CDR1 comprising the amino acid sequence of SEQ ID NO: 10; a light chain CDR2 comprising the amino acid sequence of SEQ ID NO: 12; and a light chain CDR3 comprising the amino acid sequence of at least one of SEQ ID NOs: 14 or 26.

The mesothelin-binding antibody or antigen-binding fragment of the present invention may comprise at least one of the following antibodies or antigen-binding fragments:
1) an antibody or antigen-binding fragment comprising a heavy chain variable region comprising a heavy chain CDR1 comprising the amino acid sequence set forth in SEQ ID NO: 3, a heavy chain CDR2 comprising the amino acid sequence set forth in SEQ ID NO: 5, and a heavy chain CDR3 comprising the amino acid sequence set forth in SEQ ID NO: 7; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 14; and
2) an antibody or antigen-binding fragment comprising a heavy chain variable region comprising a heavy chain CDR1 comprising the amino acid sequence set forth in SEQ ID NO: 17, a heavy chain CDR2 comprising the amino acid sequence set forth in SEQ ID NO: 19, and a heavy chain CDR3 comprising the amino acid sequence set forth in SEQ ID NO: 21; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 26.

**[Table 1]**

| Clone | Classification | | Sequence information | SEQ ID NO. |
|---|---|---|---|---|
| #19 | VH | HCDR1 | GYSFTDYN | 3 |
| | | HCDR2 | INPNYGTT | 5 |
| | | HCDR3 | AKGDYDGAGFAY | 7 |
| | VL | LCDR1 | SSVSY | 10 |
| | | LCDR2 | DTS | 12 |
| | | LCDR3 | QQWSSNPLT | 14 |
| #45 | VH | HCDR1 | GYTFTSYW | 17 |
| | | HCDR2 | IHPNSGGT | 19 |
| | | HCDR3 | ARYYYGNYVWYFDV | 21 |
| | VL | LCDR1 | SSVSY | 10 |
| | | LCDR2 | DTS | 12 |
| | | LCDR3 | QQWSSYPLT | 26 |

The mesothelin-binding antibody or antigen-binding fragment thereof according to the present invention may comprise a heavy chain variable region comprising a heavy chain FR1 comprising the amino acid sequence of at least one of SEQ ID NOs: 2 or 16; a heavy chain FR2 comprising the amino acid sequence of at least one of SEQ ID NOs: 4 or 18; a heavy chain FR3 comprising the amino acid sequence of at least one of SEQ ID NOs: 6 or 20; and a heavy chain FR4 comprising the amino acid sequence of at least one of SEQ ID NOs: 8 or 22, shown in Table 2 below.

The mesothelin-binding antibody or antigen-binding fragment thereof according to the present invention may comprise a light chain variable region comprising a light chain FR1 comprising the amino acid sequence of at least one of SEQ ID NOs: 9 or 23; a light chain FR2 comprising the amino acid sequence of at least one of SEQ ID NOs: 11 or 24; a light chain FR3 comprising the amino acid sequence of at least one of SEQ ID NOs: 13 or 25; and a light chain FR4 comprising the amino acid sequence of at least one of SEQ ID NOs: 15 or 27, shown in Table 2 below.

The mesothelin-binding antibody or antigen-binding fragment thereof according to the present invention may comprise a heavy chain variable region comprising a heavy chain FR1 comprising the amino acid sequence of at least one of SEQ ID NOs: 2 or 16; a heavy chain FR2 comprising the amino acid sequence of at least one of SEQ ID NOs: 4 or 18; a heavy chain FR3 comprising the amino acid sequence of at least one of SEQ ID NOs: 6 or 20; and a heavy chain FR4 comprising the amino acid sequence of at least one of SEQ ID NOs: 8 or 22, and comprise a light chain variable region comprising a light chain FR1 comprising the amino acid sequence of at least one of SEQ ID NOs: 9 or 23; a light chain FR2 comprising the amino acid sequence of at least one of SEQ ID NOs: 11 or 24; a light chain FR3 comprising the amino acid sequence of at least one of SEQ ID NOs: 13 or 25; and a light chain FR4 comprising the amino acid sequence of at least one of SEQ ID NOs: 15 or 27.

**[Table 2]**

| Clone | Classification | | Sequence information | SEQ ID NO. |
|---|---|---|---|---|
| #19 | VH | HFR1 | QVQLQQSGPELVKPGASVKISCKAS | 2 |
| | | HFR2 | MNWVKQSNGKSLEWIGV | 4 |
| | | HFR3 | SYNQKFKGKATITADTSSNTAYLQLSSLTSE DSAVYYC | 6 |
| | | HFR4 | WGQGTLVTVSA | 8 |
| | VL | LFR1 | DIVITQSPAIMSASPGEKVTMTCSAS | 9 |
| | | LFR2 | MHWYQQKSGTSPKRWIY | 11 |
| | | LFR3 | KLASGVPARFSGSGSGTSYSLTISSMEAEDA ATYYC | 13 |
| | | LFR4 | FGAGTKLEIKR | 15 |
| #45 | VH | HFR1 | QVQLQQPGAELVKPGASVKLSCKAS | 16 |
| | | HFR2 | MHWVKQRPGQGLEWIGM | 18 |
| | | HFR3 | NYNEKFKSKATLTVDKSSSTAYMQLSSLTS EDSAVYYC | 20 |
| | | HFR4 | WGTGTTVTVSA | 22 |
| | VL | LFR1 | DIVLTQSPAIMSTSPGEKVTMTCTAS | 23 |
| | | LFR2 | MHWYQQKSGTSPKRWIY | 24 |
| | | LFR3 | KLASGVPARFSGSGSGTSYSLTISRMEAEDA ATYYC | 25 |
| | | LFR4 | FGAGTKLEMKR | 27 |

The mesothelin-binding antibody or antigen-binding fragment thereof according to the present invention may comprise a heavy chain variable region comprising a heavy chain FR1 comprising the amino acid sequence of at least one of SEQ ID NOs: 2 or 16, a heavy chain CDR1 comprising the amino acid sequence of at least one of SEQ ID NOs: 3 or 17, a heavy chain FR2 comprising the amino acid sequence of at least one of SEQ ID NOs: 4 or 18, a heavy chain CDR2 comprising the amino acid sequence of at least one of SEQ ID NOs: 5 or 19, a heavy chain FR3 comprising the amino acid sequence of at least one of SEQ ID NOs: 6 or 20, a heavy chain CDR3 comprising the amino acid sequence of at least one of SEQ ID NOs: 7 or 21, and a heavy chain FR4 comprising the amino acid sequence of at least one of SEQ ID NOs: 8 or 22.

The mesothelin-binding antibody or antigen-binding fragment thereof according to the present invention may comprise a light chain variable region comprising a light chain FR1 comprising the amino acid sequence of at least one of SEQ ID NOs: 9 or 23, a light chain CDR1 comprising the amino acid sequence set forth in SEQ ID NO: 10, a light chain FR2 comprising the amino acid sequence of at least one of SEQ ID NOs: 11 or 24, a light chain CDR2 comprising the amino acid sequence set forth in SEQ ID NO: 12, a light chain FR3 comprising the amino acid sequence of at least one of SEQ ID NOs: 13 or 25, a light chain CDR3 comprising the amino acid sequence of at least one of SEQ ID NOs: 14 or 26, and a light chain FR4 comprising the amino acid sequence of at least one of SEQ ID NOs: 15 or 27.

The mesothelin-binding antibody or antigen-binding fragment thereof according to the present invention may comprise a heavy chain variable region comprising a heavy chain FR1 comprising the amino acid sequence of at least one of SEQ ID NOs: 2 or 16,a heavy chain CDR1 comprising the amino acid sequence of at least one of SEQ ID NOs: 3 or 17, a heavy chain FR2 comprising the amino acid sequence of at least one of SEQ ID NOs: 4 or 18, a heavy chain CDR2 comprising the amino acid sequence of at least one of SEQ ID NOs: 5 or 19, a heavy chain FR3 comprising the amino acid sequence of at least one of SEQ ID NOs: 6 or 20, a heavy chain CDR3 comprising the amino acid sequence of at least one of SEQ ID NOs: 7 or 21, and a heavy chain FR4 comprising the amino acid sequence of at least one of SEQ ID NOs: 8 or 22; and a light chain variable region comprising a light chain FR1 comprising the amino acid sequence of at least one of SEQ ID NOs: 9 or 23, a light chain CDR1 comprising the amino acid sequence set forth in SEQ ID NO: 10, a light chain FR2 comprising the amino acid sequence of at least one of SEQ ID NOs: 11 or 24, a light chain CDR2 comprising the amino acid sequence set forth in SEQ ID NO: 12, a light chain FR3 comprising the amino acid sequence of at least one of SEQ ID NOs: 13 or 25, a light chain CDR3 comprising the amino acid sequence of at least one of SEQ ID NOs: 14 or 26, and a light chain FR4 comprising the amino acid sequence of at least one of SEQ ID NO: 15 or 27.

The mesothelin-binding antibody or antigen-binding fragment of the present invention may comprise at least one of the following antibodies or antigen-binding fragments:
3) an antibody or antigen-binding fragment comprising a heavy chain variable region comprising a heavy chain FR1 comprising the amino acid sequence set forth in SEQ ID NO: 2, a heavy chain CDR1 comprising the amino acid sequence set forth in SEQ ID NO: 3, a heavy chain FR2 comprising the amino acid sequence set forth in SEQ ID NO: 4, a heavy chain CDR2 comprising the amino acid sequence set forth in SEQ ID NO: 5, a heavy chain FR3 comprising the amino acid sequence set forth in SEQ ID NO: 6, a heavy chain CDR3 comprising the amino acid sequence set forth in SEQ ID NO: 7, and a heavy chain FR4 comprising the amino acid sequence set forth in SEQ ID NO: 8; and a light chain variable region comprising a light chain FR1 comprising the amino acid sequence set forth in SEQ ID NO: 9, a light chain CDR1 comprising the amino acid sequence set forth in SEQ ID NO: 10, a light chain FR2 comprising the amino acid sequence set forth in SEQ ID NO: 11, a light chain CDR2 comprising the amino acid sequence set forth in SEQ ID NO: 12, a light chain FR3 comprising the amino acid sequence set forth in SEQ ID NO: 13, a light chain CDR3 comprising the amino acid sequence set forth in SEQ ID NO: 14, and a light chain FR4 comprising the amino acid sequence set forth in SEQ ID NO: 15; and
4) an antibody or antigen-binding fragment comprising a heavy chain variable region comprising a heavy chain FR1 comprising the amino acid sequence set forth in SEQ ID NO: 16, a heavy chain CDR1 comprising the amino acid sequence set forth in SEQ ID NO: 17, a heavy chain FR2 comprising the amino acid sequence set forth in SEQ ID NO: 18, a heavy chain CDR2 comprising the amino acid sequence set forth in SEQ ID NO: 19, a heavy chain FR3 comprising the amino acid sequence set forth in SEQ ID NO: 20, a heavy chain CDR3 comprising the amino acid sequence set forth in SEQ ID NO: 21, and a heavy chain FR4 comprising the amino acid sequence set forth in SEQ ID NO: 22; and a light chain variable region comprising a light chain FR1 comprising the amino acid sequence set forth in SEQ ID NO: 23, a light chain CDR1 comprising the amino acid sequence set forth in SEQ ID NO: 10, a light chain FR2 comprising the amino acid sequence set forth in SEQ ID NO: 24, a light chain CDR2 comprising the amino acid sequence set forth in SEQ ID NO: 12, a light chain FR3 comprising the amino acid sequence set forth in SEQ ID NO: 25, a light chain CDR3 comprising the amino acid sequence set forth in SEQ ID NO: 26, and a light chain FR4 comprising the amino acid sequence set forth in SEQ ID NO: 27.

The mesothelin-binding antibody or antigen-binding fragment of the present invention may comprise a heavy chain variable region comprising the amino acid sequence of at least one of SEQ ID NOs: 38 or 42; and a light chain variable region comprising the amino acid sequence of at least one of SEQ ID NOs: 39 or 43, shown in Table 3 below.

The mesothelin-binding antibody or antigen-binding fragment of the present invention may comprise at least one of the following antibodies or antigen-binding fragments:
5) an antibody or antigen-binding fragment comprising a heavy chain variable region comprising the amino acid sequence set forth in SEQ ID NO: 38, and a light chain variable region comprising the amino acid sequence set forth in SEQ ID NO: 39; and
6) an antibody or antigen-binding fragment comprising a heavy chain variable region comprising the amino acid sequence set forth in SEQ ID NO: 42, and a light chain variable region comprising the amino acid sequence set forth in SEQ ID NO: 43.

**[Table 3]**

| Clone | Classification | Sequence information | SEQ ID NO. |
|---|---|---|---|
| #19 | VH | QVQLQQSGPELVKPGASVKISCKASGYSFTDY NMNWVKQSNGKSLEWIGVINPNYGTTSYNQ KFKGKATITADTSSNTAYLQLSSLTSEDSAVY YCAKGDYDGAGFAYWGQGTLVTVSA | 38 |
| | VL | DIVITQSPAIMSASPGEKVTMTCSASSSVSYM HWYQQKSGTSPKRWIYDTSKLASGVPARFSG SGSGTSYSLTISSMEAEDAATYYCQQWSSNPL TFGAGTKLEIKR | 39 |
| #45 | VH | QVQLQQPGAELVKPGASVKLSCKASGYTFTS YWMHWVKQRPGQGLEWIGMIHPNSGGTNY | 42 |
| | | NEKFKSKATLTVDKSSSTAYMQLSSLTSEDSA VYYCARYYYGNYVWYFDVWGTGTTVTVSA | |
| | VL | DIVLTQSPAIMSTSPGEKVTMTCTASSSVSYM HWYQQKSGTSPKRWIYDTSKLASGVPARFSG SGSGTSYSLTISRMEAEDAATYYCQQWSSYPL TFGAGTKLEMKR | 43 |

In one embodiment, the antibody provided by the present invention may be a monoclonal antibody.

In one embodiment, the antigen-binding fragment provided by the present invention may be scFv.

The antibody or antigen-binding fragment of the present invention may further comprise a linker between the heavy chain variable region and the light chain variable region. Here, the linker may be a peptide linker and may be about 10-25 aa in length. For example, the linker may include hydrophilic amino acids such as glycine and/or serine, without being limited thereto.

Specifically, the linker in the present invention may comprise, for example, (GS)n, (GGS)n, (GSGGS)n or (GnS)m (n and m are each an integer ranging from 1 to 10), preferably GGGGS, more preferably GGGGSGGGGSGGGGS represented by SEQ ID NO: 50, without being limited thereto.

The mesothelin-binding antigen-binding fragment of the present invention may be an scFv and may comprise the amino acid sequence of at least one of SEQ ID NOs: 51 or 53.

The antibody or antigen-binding fragment of the present invention may include not only the sequence of the anti-mesothelin antibody or antigen-binding fragment of the present invention described herein, but also biological equivalents thereof, as long as it is capable of specifically recognizing mesothelin. For example, additional modifications may be made to the amino acid sequence of the antibody in order to further improve the binding affinity and/or other biological properties of the antibody. Such modifications include, for example, deletions from, and/or insertions into and/or substitutions of residues within the amino acid sequence of the antibody or antigen-binding fragment. Such amino acid variations are based on the relative similarity of amino acid side-chain substituents, for example, their hydrophobicity, hydrophilicity, charge, size, and the like. An analysis of the size, shape and type of the amino acid side-chain substituents reveals that arginine, lysine and histidine are all positively charged residues; that alanine, glycine and serine all have a similar size; and that phenylalanine, tryptophan and tyrosine all have a similar shape. Therefore, based upon these considerations, arginine, lysine and histidine; alanine, glycine and serine; and phenylalanine, tryptophan and tyrosine; may be defined as biologically functional equivalents.

Considering the above-described variations having biological equivalent activity, the antibody or antigen-binding fragment of the present invention or the nucleic acid molecule encoding the same is interpreted to also include a sequence having substantial identity with the sequence shown in the sequence listing. "Substantial identity" refers to an amino acid sequence having at least 90%, preferably at least 95%, at least 96%, at least 97%, at least 98%, or at least 99% homology, as determined by aligning any other sequence with the sequence of the present invention to correspond to each other as much as possible and analyzing the aligned sequence using an algorithm commonly used in the art. Alignment methods for sequence comparison are well-known in the art. The NCBI Basic Local Alignment Search Tool (BLAST) is accessible through NCBI or the like, and may be used in conjunction with sequence analysis programs, such as blastp, blasm, blastx, tblastn and tblastx, on the Internet. BLAST is available from http://www.ncbi.nlm.nih.gov/BLASTI. Sequence homology comparison methods using this program can be identified online (http://www.ncbi.nlm.nih.gov/BLAST/blast_ help.html).

Based thereon, the antibody or antigen-binding fragment of the present invention may have 90%, 91%, 92%, 93%, 94%, 95%, 96%, 97%, 98%, 99%, or higher homology with a specified sequence or all of the sequences described in the present specification. Such homology may be determined by sequence comparison and/or alignment using methods known in the art. For example, the percent sequence homology of the nucleic acid or protein of the present invention may be determined using a sequence comparison algorithm (i.e., BLAST or BLAST 2.0), manual alignment, or visual inspection.

The binding affinity of the anti-mesothelin antibody or antigen-binding fragment provided by the present invention to mesothelin is in the range of 10⁻⁵ M to 10⁻¹² M. For example, the binding affinity of the anti-mesothelin antibody or antigen-binding fragment to mesothelin may be 10⁻⁵ M to 10⁻¹² M, 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M, without being limited thereto.

### 2. Humanized Antibody or Antigen-Binding Fragment Thereof

The present invention provides a mesothelin-binding humanized antibody or an antigen-binding fragment thereof.

The humanized antibody provided by the present invention is able to specifically bind to mesothelin and may have low immunogenicity even when administered to the human body.

The humanized antibody or antigen-binding fragment thereof according to the present invention is produced based on a mouse antibody comprising a heavy chain variable region and light chain variable region having the amino acid sequences shown in Tables 1 to 3 above.

The sequence of the heavy chain variable region of the humanized antibody or antigen-binding fragment thereof according to the present invention may be at least 70%, 75%, 80%, 85%, 90%, 91%, 92%, 93%, 94%, 95%, 96%, 97%, 98% or 99% identical to the amino acid sequence set forth SEQ ID NO: 38.

The sequence of the light chain variable region of the humanized antibody or antigen-binding fragment thereof according to the present invention may be at least 70%, 75%, 80%, 85%, 90%, 91%, 92%, 93%, 94%, 95%, 96%, 97%, 98% or 99% identical to the amino acid sequence set forth SEQ ID NO: 39.

The mesothelin-binding humanized antibody or antigen-binding fragment thereof according to the present invention may comprise a heavy chain variable region comprising a heavy chain CDR1 comprising the amino acid sequence set forth in SEQ ID NO: 29; a heavy chain CDR2 comprising the amino acid sequence set forth in SEQ ID NO: 5; and a heavy chain CDR3 comprising the amino acid sequence set forth in SEQ ID NO: 32, shown in Table 4 below.

In addition, the mesothelin-binding humanized antibody or antigen-binding fragment thereof according to the present invention may comprise a light chain variable region comprising a light chain CDR1 comprising the amino acid sequence set forth in SEQ ID NO: 10; a light chain CDR2 comprising the amino acid sequence set forth in SEQ ID NO: 12; and a light chain CDR3 comprising the amino acid sequence set forth in SEQ ID NO: 14, shown in Table 4 below.

The mesothelin-binding humanized antibody or antigen-binding fragment thereof according to the present invention may comprise a heavy chain variable region comprising a heavy chain CDR1 comprising the amino acid sequence set forth in SEQ ID NO: 29, a heavy chain CDR2 comprising the amino acid sequence set forth in SEQ ID NO: 5, and a heavy chain CDR3 comprising the amino acid sequence set forth in SEQ ID NO: 32; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 14.

**[Table 14]**

| Classification | | Sequence information | SEQ ID NO. |
|---|---|---|---|
| VH | HCDR1 | GYTFTDYN | 29 |
| | HCDR2 | INPNYGTT | 5 |
| | HCDR3 | ARGDYDGAGFAY | 32 |
| VL | LCDR1 | SSVSY | 10 |
| | LCDR2 | DTS | 12 |
| | LCDR3 | QQWSSNPLT | 14 |

The mesothelin-binding humanized antibody or antigen-binding fragment thereof according to the present invention may comprise a heavy chain variable region comprising a heavy chain FR1 comprising the amino acid sequence set forth in SEQ ID NO: 28; a heavy chain FR2 comprising the amino acid sequence set forth in SEQ ID NO: 30; a heavy chain FR3 comprising the amino acid sequence set forth in SEQ ID NO: 31; and a heavy chain FR4 comprising the amino acid sequence shown in SEQ ID NO: 33, shown in Table 5 below.

In addition, the mesothelin-binding humanized antibody or antigen-binding fragment thereof according to the present invention may comprise a light chain variable region comprising a light chain FR1 comprising the amino acid sequence set forth in SEQ ID NO: 34; a light chain FR2 comprising the amino acid sequence set forth in SEQ ID NO: 35; a light chain FR3 comprising the amino acid sequence set forth in SEQ ID NO: 36; and a light chain FR4 comprising the amino acid sequence set forth in SEQ ID NO: 37, shown in Table 5 below.

The mesothelin-binding humanized antibody or antigen-binding fragment thereof according to the present invention may comprise a heavy chain variable region comprising a heavy chain FR1 comprising the amino acid sequence set forth in SEQ ID NO: 28, a heavy chain FR2 comprising the amino acid sequence set forth in SEQ ID NO: 30, a heavy chain FR3 comprising the amino acid sequence set forth in SEQ ID NO: 31, and a heavy chain FR4 comprising the amino acid sequence shown in SEQ ID NO: 33; and a light chain variable region comprising a light chain FR1 comprising the amino acid sequence set forth in SEQ ID NO: 34, a light chain FR2 comprising the amino acid sequence set forth in SEQ ID NO: 35; a light chain FR3 comprising the amino acid sequence set forth in SEQ ID NO: 36; and a light chain FR4 comprising the amino acid sequence set forth in SEQ ID NO: 37.

**[Table 5]**

| Classification | | Sequence information | SEQ ID NO. |
|---|---|---|---|
| VH | HFR1 | QVQLVQSGAEVKKPGASVKVSCKAS | 28 |
| | HFR2 | MNWVRQAPGQGLEWMGV | 30 |
| | HFR3 | SYNQKFKGRVTMTRDTSTSTVYMELSSLRSE DTAVYYC | 31 |
| | HFR4 | WGQGTLVTVSS | 33 |
| VL | LFR1 | EIVLTQSPATLSLSPGERATLSCSAS | 34 |
| | LFR2 | MHWYQQKPGQAPRLLIY | 35 |
| | LFR3 | KLASGIPARFSGSGSGTDFTLTISSLEPEDFAV YYC | 36 |
| | LFR4 | FGQGTKLEIKR | 37 |

The mesothelin-binding humanized antibody or antigen-binding fragment thereof according to the present invention may comprise a heavy chain variable region comprising a heavy chain FR1 comprising the amino acid sequence set forth in SEQ ID NO: 28, a heavy chain CDR1 comprising the amino acid sequence set forth in SEQ ID NO: 29, a heavy chain FR2 comprising the amino acid sequence set forth in SEQ ID NO: 30, a heavy chain CDR2 comprising the amino acid sequence set forth in SEQ ID NO: 5, a heavy chain FR3 comprising the amino acid sequence set forth in SEQ ID NO: 31, a heavy chain CDR3 comprising the amino acid sequence set forth in SEQ ID NO: 32, and a heavy chain FR4 comprising the amino acid sequence set forth in SEQ ID NO: 33.

The mesothelin-binding humanized antibody or antigen-binding fragment thereof according to the present invention may comprise a light chain variable region comprising a light chain FR1 comprising the amino acid sequence set forth in SEQ ID NO: 34, a light chain CDR1 comprising the amino acid sequence set forth in SEQ ID NO: 10, a light chain FR2 comprising the amino acid sequence set forth in SEQ ID NO: 35, a light chain CDR2 comprising the amino acid sequence set forth in SEQ ID NO: 12, a light chain FR3 comprising the amino acid sequence set forth in SEQ ID NO: 36, a light chain CDR3 comprising the amino acid sequence set forth in SEQ ID NO: 14, and a light chain FR4 comprising the amino acid sequence set forth in SEQ ID NO: 37.

The mesothelin-binding humanized antibody or antigen-binding fragment thereof according to the present invention may comprise a heavy chain variable region comprising a heavy chain FR1 comprising the amino acid sequence set forth in SEQ ID NO: 28, a heavy chain CDR1 comprising the amino acid sequence set forth in SEQ ID NO: 29, a heavy chain FR2 comprising the amino acid sequence set forth in SEQ ID NO: 30, a heavy chain CDR2 comprising the amino acid sequence set forth in SEQ ID NO: 5, a heavy chain FR3 comprising the amino acid sequence set forth in SEQ ID NO: 31, a heavy chain CDR3 comprising the amino acid sequence set forth in SEQ ID NO: 32, and a heavy chain FR4 comprising the amino acid sequence set forth in SEQ ID NO: 33, and comprise a light chain variable region comprising a light chain FR1 comprising the amino acid sequence set forth in SEQ ID NO: 34, a light chain CDR1 comprising the amino acid sequence set forth in SEQ ID NO: 10, a light chain FR2 comprising the amino acid sequence set forth in SEQ ID NO: 35, a light chain CDR2 comprising the amino acid sequence set forth in SEQ ID NO: 12, a light chain FR3 comprising the amino acid sequence set forth in SEQ ID NO: 36, a light chain CDR3 comprising the amino acid sequence set forth in SEQ ID NO: 14, and a light chain FR4 comprising the amino acid sequence set forth in SEQ ID NO: 37.

In addition, the mesothelin-binding humanized antibody or antigen-binding fragment thereof according to the present invention may comprise a heavy chain variable region comprising the amino acid sequence set forth in SEQ ID NO: 46, and a light chain variable region comprising the amino acid sequence set forth in SEQ ID NO: 47, shown in Table 6 below.

**[Table 6]**

| Sequence information | | SEQ ID NO. |
|---|---|---|
| VH | QVQLVQSGAEVKKPGASVKVSCKASGYTFTDYN MNWVRQAPGQGLEWMGVINPNYGTTSYNQKFKGRVTM TRDTSTSTVYMELSSLRSEDTAVYYCARGDYDGAGFAYW GQGTLVTVSS | 46 |
| VL | EIVLTQSPATLSLSPGERATLSCSASSSVSYMHWYQQKPG QAPRLLIYDTSKLASGIPARFSGSGSGTDFTLTISSLEPEDFA VYYCQQWSSNPLTFGQGTKLEIKR | 47 |

In one embodiment, the humanized antibody provided by the present invention may be a monoclonal antibody.

In one embodiment, the antigen-binding fragment provided by the present invention may be an scFv.

The humanized antibody or antigen-binding fragment thereof according to the present invention may further comprise a linker between the heavy chain variable region and the light chain variable region. Here, the linker may be a peptide linker and may be about 10-25 aa in length. For example, the linker may include hydrophilic amino acids such as glycine and/or serine, without being limited thereto. Specifically, the linker in the present invention may comprise, for example, (GS)n, (GGS)n, (GSGGS)n or (GnS)m (n and m are each an integer ranging from 1 to 10), preferably GGGGS, more preferably GGGGSGGGGSGGGGS represented by SEQ ID NO: 50, without being limited thereto.

The mesothelin-binding humanized antigen-binding fragment of the present invention may be an scFv, wherein the sequence of the scFv may be at least 70%, 75%, 80%, 85%, 90%, 91%, 92%, 93%, 94%, 95%, 96%, 97%, 98% or 99% identical to the amino acid sequence set forth in SEQ ID NO: 51.

In addition, the mesothelin-binding antigen-binding fragment of the present invention may be an scFv comprising the amino acid sequence of SEQ ID NO: 55.

**[Table 7]**

| Sequence information | | SEQ ID NO. |
|---|---|---|
| scFv | EIVLTQSPATLSLSPGERATLSCSASSSVSYMHW YQQKPGQAPRLLIYDTSKLASGIPARFSGSGSGT | 55 |
| | DFTLTISSLEPEDFAVYYCQQWSSNPLTFGQGTK LEIKRGGGGSGGGGSGGGGSQVQLVQSGAEVK KPGASVKVSCKASGYTFTDYNMNWVRQAPGQ GLEWMGVINPNYGTTSYNQKFKGRVTMTRDTS TSTVYMELSSLRSEDTAVYYCARGDYDGAGFA YWGQGTLVTVSS | |

The humanized antibody or antigen-binding fragment thereof according to the present invention may include not only the sequence of the humanized anti-mesothelin antibody or antigen-binding fragment of the present invention described herein, but also biological equivalents thereof, as long as it is capable of specifically recognizing mesothelin. The biological equivalents are as described above, and thus the description thereof will be omitted below to avoid excessive complexity of the specification.

The binding affinity of the mesothelin-binding humanized antibody or antigen-binding fragment provided by the present invention to mesothelin is in the range of 10⁻⁵ M to 10⁻¹² M. For example, the binding affinity of the mesothelin-binding humanized antibody or antigen-binding fragment to mesothelin may be 10⁻⁵ M to 10⁻¹² M, 10⁻⁶ M to 10⁻¹² M, 10⁻⁷ M to 10⁻¹² M, 10⁻⁸ M to 10⁻¹² M, 10⁻⁹ M to 10⁻¹² M, 10⁻⁵ M to 10⁻¹¹ M, 10⁻⁶ M to 10⁻¹¹ M, 10⁻⁷ M to 10⁻¹¹ M, 10⁻⁸ M to 10⁻¹¹ M, 10⁻⁹ M to 10⁻¹¹ M, 10⁻¹⁰ M to 10⁻¹¹ M, 10⁻⁵ M to 10⁻¹⁰ M, 10⁻⁶ M to 10⁻¹⁰ M, 10⁻⁷ M to 10⁻¹⁰ M, 10⁻⁸ M to 10⁻¹⁰ M, 10⁻⁹ M to 10⁻¹⁰ M, 10⁻⁵ M to 10⁻⁹ M, 10⁻⁶ M to 10⁻⁹ M, 10⁻⁷ M to 10⁻⁹ M, 10⁻⁸ M to 10⁻⁹ M, 10⁻⁵ M to 10⁻⁸ M, 10⁻⁶ M to 10⁻⁸ M, 10⁻⁷ M to 10⁻⁸ M, 10⁻⁵ M to 10⁻⁷ M, 10⁻⁶ M to 10⁻⁷ M, or 10⁻⁵ M to 10⁻⁶ M, without being limited thereto.

The binding affinity of the mesothelin-binding humanized antibody or antigen-binding fragment provided by the present invention to mesothelin antigen may be equal to or higher than that of a mouse antibody defined in Table 3 above, or an antigen-binding fragment (e.g., scFv) comprising the amino acid sequence of SEQ ID NO: 51.

According to another embodiment of the present invention, the present invention relates to a nucleic acid molecule encoding an antibody (including humanized antibody) or antigen-binding fragment provided by the present invention.

As used herein, the term "nucleic acid" is meant to encompass DNA (gDNA and cDNA) and RNA molecules, and nucleotides, which are the basic structural units in nucleic acid, include not only natural nucleotides, but also analogues having modified sugar or base moieties. The sequences of nucleic acids encoding the heavy and light chain variable regions of the present invention may be modified. The modifications include additions, deletions, or non-conservative or conservative substitutions of nucleotides.

In the present invention, the DNA encoding the antibody or antigen-binding fragment may be easily isolated or synthesized using conventional molecular biology techniques (e.g., by using oligonucleotide probes that are capable of binding specifically to DNA encoding the heavy and light chains of the antibody). The nucleic acid is isolated and inserted into a replicable vector for further cloning (DNA amplification) or further expression.

As an example, the nucleic acid molecule of the present invention may comprise the nucleotide sequence set forth in SEQ ID NO: 40, which encodes the heavy chain variable region set forth in SEQ ID NO: 38.

As an example, the nucleic acid molecule of the present invention may comprise the nucleotide sequence set forth in SEQ ID NO: 44, which encodes the heavy chain variable region set forth in SEQ ID NO: 42.

As an example, the nucleic acid molecule of the present invention may comprise the nucleotide sequence set forth in SEQ ID NO: 48, which encodes the heavy chain variable region set forth in SEQ ID NO: 46.

As an example, the nucleic acid molecule of the present invention may comprise the nucleotide sequence set forth in SEQ ID NO: 41, which encodes the light chain variable region set forth in SEQ ID NO: 39.

As an example, the nucleic acid molecule of the present invention may comprise the nucleotide sequence set forth in SEQ ID NO: 45, which encodes the light chain variable region set forth in SEQ ID NO: 43.

As an example, the nucleic acid molecule of the present invention may comprise the nucleotide sequence set forth in SEQ ID NO: 49, which encodes the heavy chain variable region set forth in SEQ ID NO: 47.

As an example, the nucleic acid molecule of the present invention may comprise the nucleotide sequence set forth in SEQ ID NO: 40, which encodes the heavy chain variable region, and the nucleotide sequence set forth in SEQ ID NO: 41, which encodes the light chain variable region.

As an example, the nucleic acid molecule of the present invention may comprise the nucleotide sequence set forth in SEQ ID NO: 44, which encodes the heavy chain variable region, and the nucleotide sequence set forth in SEQ ID NO: 45, which encodes the light chain variable region.

As an example, the nucleic acid molecule of the present invention may comprise the nucleotide sequence set forth in SEQ ID NO: 48, which encodes the heavy chain variable region, and the nucleotide sequence set forth in SEQ ID NO: 49, which encodes the light chain variable region.

As an example, the nucleic acid molecule of the present invention may comprise the nucleotide sequence set forth in SEQ ID NO: 52, which encodes the scFv set forth in SEQ ID NO: 51.

As an example, the nucleic acid molecule of the present invention may comprise the nucleotide sequence set forth in SEQ ID NO: 54, which encodes the scFv set forth in SEQ ID NO: 53.

As an example, the nucleic acid molecule of the present invention may comprise the nucleotide sequence set forth in SEQ ID NO: 56, which encodes the scFv set forth in SEQ ID NO: 55.

According to another embodiment, the present invention relates to a recombinant expression vector containing the nucleic acid molecule provided by the present invention.

In the present invention, the "vector" refers to a recombinant vector that can be transfected into a suitable host cell to express a target protein, and means a gene construct that contains essential regulatory elements operably linked so that an inserted gene is expressed. As used herein, "operably linked" means that a nucleic acid expression regulatory sequence and a nucleic acid sequence encoding a protein of interest are functionally linked to perform a general function. Operational linkage with a recombinant vector can be made using recombinant DNA techniques well known in the art, and site-specific DNA cleavage and linkage can be easily achieved using enzymes commonly known in the art.

In the present invention, as a recombinant expression vector for insertion of a foreign gene, various types of vectors, such as nanoparticles, plasmids, viruses, and cosmids, may be used. The type of recombinant vector is not particularly limited as long as the recombinant vector functions to express a desired gene and produce a desired protein in various types of prokaryotic and eukaryotic host cells. However, particularly, it is possible to use a vector capable of producing a large amount of a foreign protein that is in a form similar to its natural state while having a promoter with strong activity and strong expression capacity.

Various gene delivery vehicles are known in the art and include both viral and non-viral (e.g., naked DNA, plasmid) vectors. Viral vectors suitable for gene transfer are known to those skilled in the art. Non-limiting examples of the viral vector include retroviral vectors (derived from Moloney murine leukemia virus vector (MoMLV), MSCV, SFFV, MPSV, SNV, etc.), lentiviral vectors (e.g. derived from HIV-1, HIV-2, SIV, BIV, FIV etc.), adenoviral (Ad) vectors including replication competent, replication deficient and gutless forms thereof, adeno-associated viral (AAV) vectors, simian virus 40 (SV-40) vectors, bovine papilloma virus vectors, Epstein-Barr virus vectors, herpes virus vectors, vaccinia virus vectors, Harvey murine sarcoma virus vectors, murine mammary tumor virus vectors, Rous sarcoma virus vectors, parvovirus vectors, poliovirus vectors, vesicular stomatitis virus vectors, Maraba virus vectors, and Group B adenovirus enadenotucirev vectors.

Non-viral vectors for gene delivery include naked DNA, plasmids, transposons, and mRNA, among others. Non-limiting examples include pKK plasmids (Clonetech), pUC plasmids, pET plasmids (Novagen, Inc., Madison, Wis.), pRSET or pREP plasmids (Invitrogen, San Diego, Calif.), and pMAL plasmids (New England Biolabs, Beverly, Mass.).

In the present invention, such vectors may be introduced into many appropriate host cells, using methods disclosed or cited herein or otherwise known to those skilled in the relevant art.

A suitable expression vector of the present invention may contain expression regulatory elements such as a promoter, initiation codon, stop codon, polyadenylation signal, or enhancer, as well as a nucleotide sequence encoding a signal peptide for membrane targeting or secretion. The initiation codon and stop codon are generally considered as a part of a nucleotide sequence encoding an immunogenic target protein, and should exhibit action in s subject when the gene construct is administered, and be in frame with a coding sequence.

As used herein, the term "promoter" refers to any sequence that regulates the expression of a coding sequence, such as a gene. Promoters may be, for example, constitutive, inducible, repressible, or tissue-specific. A "promoter" is a control sequence that is a region of a polynucleotide sequence at which initiation and rate of transcription are controlled. In the present invention, non-limiting examples of the promoter include Rous sarcoma virus (RSV) LTR promoter (optionally with the RSV enhancer), Cytomegalovirus (CMV) promoter, SV40 promoter, dihydrofolate reductase promoter, β-actin promoter, phosphoglycerol kinase (PGK) promoter, U6 promoter, EF1 alpha short (EFS) promoter, human polypeptide chain elongation factor (EF1a) promoter, P5 promoter, Ubc promoter, CAG promoter, TRE promoter, UAS promoter, Ac5 promoter, polyhedrin promoter, CaMKIIa promoter, Gal1 promoter, TEF1 promoter, GDS promoter, ADH1 promoter, CaMV35S promoter, ubiquitin (Ubi) promoter, such as ubiquitin C (UbiC), H1 promoter, U6 promoter, alpha-1-antitrypsin promoter, spleen focus-forming virus (SFFV) promoter, and the like.

In addition, in the present invention, the promoter may be coupled to an enhancer to increase transcription efficiency. Here, non-limiting examples of the enhancer may include, but are not limited to, an RSV enhancer, a CMV enhancer, or an α-fetoprotein MERII enhancer. The vector of the present invention may be fused with another sequence to facilitate purification of the antibody expressed therefrom. Examples of the sequence to be fused include, but are not limited to, glutathione S-transferase (Pharmacia, USA), maltose binding protein (NEB, USA), FLAG (IBI, USA), and 6x His (hexahistidine; Quiagen, USA).

The vector of the present invention contains, as a selectable marker, an antibiotic resistance gene commonly used in the art. Examples of the antibiotic resistance gene include, but are not limited to, resistance genes against ampicillin, gentamicin, carbenicillin, chloramphenicol, streptomycin, kanamycin, geneticin, neomycin and tetracycline.

According to another embodiment of the present invention, the present invention relates to a host cell transfected with the expression vector provided by the present invention.

As used herein, the term "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as *E. coli, Streptomyces, Salmonella typhimurium;* fungal cells such as yeast cells and *Pichia pastoris;* insect cells such as *Drosophila* and *Spodoptera* Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and it is possible to use any cell known to those skilled in the art which can be used as a host cell.

In the present invention, the transfection method is any method of introducing the desired vector into the host cell, and may include any known method capable of introducing the vector into the host cell. Examples of the transfection method include, but are not limited to, a method using CaCl₂, electroporation, microinjection, calcium phosphate precipitation, electroporation, liposome-mediated transfection, DEAE-dextran treatment, gene bombardment, and transfection using viruses.

According to another embodiment, the present invention relates to a method for producing an antibody or antigen-binding fragment that binds specifically to mesothelin, the method comprising steps of: producing the antibody (including humanized antibody) or antigen-binding fragment according to the present invention by culturing the host cells; and isolating and purifying the produced antibody.

In the present invention, the host cells may be cultured in various media. Any commercially available medium may be used as a culture medium without limitation. All other necessary supplements known to those skilled in the art may also be contained at suitable concentrations. Culture conditions, such as temperature, pH, etc., have already been used with host cells selected for expression, which will be apparent to those skilled in the art.

In the present invention, recovery of the antibody or antigen-binding fragment thereof may be performed, for example, by removing impurities through centrifugation or ultrafiltration, and purifying the resulting material using, for example, affinity chromatography. Additional other purification techniques may be used, such as anion or cation exchange chromatography, hydrophobic interaction chromatography, hydroxylapatite chromatography, etc.

According to another embodiment of the present invention, the present invention relates to a bi- or multi-specific antibody comprising the antibody (including humanized antibody) or antigen-binding fragment provided by the present invention.

As used herein, the term "bispecific antibody" refers to an antibody having a binding affinity or antagonism to one or more targets, and means a form in which antibodies having a binding affinity or antagonism to two different targets are linked, or an antibody having a binding affinity to one target and a substance having an antagonism to the other target are linked.

As used herein, the term "multispecific antibody" refers to an antibody having binding specificity for at least three different antigens. The multispecific antibody may include a tri- or higher specific antibody, for example, a trispecific antibody, a tetraspecific antibody, or an antibody that targets 5 or more targets.

In the present invention, antibodies belonging to bispecific or multispecific antibodies may be classified into scFv-based antibodies, Fab-based antibodies, and IgG-based antibodies. A bispecific or multispecific antibody can inhibit or enhance two or more signals at the same time, and thus may be more effective than inhibiting/amplifying one signal. Compared to the case where each signal is treated with each signal inhibitor, the bispecific or multispecific antibody may be administered at a low dose and inhibit/enhance two or more signals in the same time and space.

In the present invention, methods for producing bispecific or multispecific antibodies are well known. Traditionally, the recombinant production of bispecific antibodies is based on the co-expression of two or more immunoglobulin heavy/light chain pairs under conditions where the two or more heavy chains have different specificities.

In the present invention, in the case of scFv-based bispecific or multispecific antibodies, a diabody having a hybrid scFv in a heterodimeric form may be prepared by combining VL and VH of different scFvs. Also, tandem ScFv may be prepared by linking different scFvs to each other. A heterodimeric miniantibody may be prepared by expressing CH1 and CL of Fab at the ends of scFv, respectively. Also, a minibody in a heterodimeric scFv form may be prepared by substituting some amino acids of the CH3 domain, which is a homodimeric domain of Fc, to change to the heterodimeric structure in a 'knob into hole' form, and expressing these modified CH3 domains at different scFv ends, respectively.

In the present invention, in the case of Fab-based bispecific or multispecific antibodies, a heterodimeric Fab may be prepared by combining individual Fabs against a specific antigen with each other using a disulfide bond or a mediator, and the antigen valency can be doubled by expressing scFvs against different antigens at the ends of the heavy or light chains of a specific Fab, or it can be prepared to have four antigen valencies in the form of homodimers by providing a hinge region between the Fab and scFv. In addition, a bispecific bibody with three antigen valencies can be prepared by fusing scFvs against different antigens to the light and heavy chain ends of the Fab, and a trispecific tribody with three antigen valencies can be prepared by fusing different scFvs to the light and heavy chain ends of the Fab, and it can also be obtained by chemically conjugating three different Fabs.

In the present invention, in the case of IgG-based bispecific or multispecific antibodies, a method of producing a bispecific antibody by hybridizing mouse and rat hybridomas to produce a hybrid hybridoma (also known as quadroma) was developed by Trion Pharma. In addition, a bispecific antibody in the so-called "Holes and Knob" form may be prepared in a heterodimeric form by modifying some amino acids of the CH3 homodimeric domain of Fc with respect to different heavy chains while sharing the light chain domain. In addition to the bispecific antibody in a heterodimeric form, (ScFv)₄-IgG in a homodimeric form may be prepared by fusion-expressing two different ScFvs in the constant domains of the light chain and the heavy chain of IgG instead of the variable domains thereof. Also, Imclone reported a bispecific antibody produced by fusing only a single variable domain for mouse platelet-derived growth factor receptor-α to the N-terminus of the light chain of a chimeric monoclonal antibody IMC-1C11 against human VEGFR-2. In addition, an antibody having multiple antigen valencies for CD20 can be prepared by the "dock and lock (DNL)" method using a dimerization and docking domain (DDD) of an R subunit of protein kinase A (PKA) and an anchoring domain of the PKA.

The present invention also includes a wide variety of recombinant antibody formats, for example, bivalent or higher, trivalent or higher, or tetravalent or higher bispecific or multispecific antibodies. A bivalent or higher, trivalent or higher, or tetravalent or higher antibody indicates that two or more binding domains, three or more binding domains, or four or more binding domains are present in the antibody molecules, respectively.

The bispecific or multispecific antibody according to the present invention may comprise the mesothelin-binding antibody or antigen-binding fragment according to the present invention, specifically in the form of an IgG complete antibody or a fragment thereof, for example, a single chain Fv, a VH domain and/or a VL domain, Fab, or (Fab)2.

In addition, the bispecific or multispecific antibody according to the present invention may comprise an antibody that binds to a target different from the target of the antibody targeting mesothelin. For example, it may comprise an antibody targeting at least one selected from the group consisting of PD-1, PD-L1, BTLA, CTLA-4, VISTA, LAG3, TIM3, CD137(4-1BB), VISTA, CD258(LIGHT), TIGIT, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, NRP1, and MARCO, specifically in the form of an IgG complete antibody or a fragment thereof, for example, a single chain Fv, a VH domain and/or a VL domain, Fab, or (Fab)2.

Additional binding specificity induced or mediated by targets other than mesothelin can be ensured through the bispecific or multispecific antibody according to the present invention.

For example, the bispecific antibody according to the present invention is able to simultaneously target mesothelin and at least one selected from the group consisting of PD-1, PD-L1, BTLA, CTLA-4, VISTA, LAG3, TIM3, CD137(4-1BB), VISTA, CD258(LIGHT), TIGIT, CD3, CD20, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, NRP1, MARCO, FcRH5, HER2, LYPD1, LY6G6D, PMEL17, LY6E, NKG2D, CD3, CD16a, CD19, CD33, CD22, CD79A, CD79B, EDAR, GFRA1, MRP4, RET, Steap1, and TenB2.

For example, the multispecific antibody according to the present invention is able to simultaneously target mesothelin and two or more selected from the group consisting of PD-1, PD-L1, BTLA, CTLA-4, VISTA, LAG3, TIM3, CD137(4-1BB), VISTA, CD258(LIGHT), TIGIT, CD3, CD20, CD134(OX40), CD28, CD278(ICOS), CD27, CD154(CD40L), CD357(GITR), CD30, DR3, CD226(DNAM1), CD96, CD200, CD200R, transferrin receptor, c-Met, EGFR, HER2, KDR, PDGFRa, NRP1, MARCO, FcRH5, HER2, LYPD1, LY6G6D, PMEL17, LY6E, NKG2D, CD3, CD16a, CD19, CD33, CD22, CD79A, CD79B, EDAR, GFRA1, MRP4, RET, Steap1, and TenB2.

According to another embodiment of the present invention, the present invention relates to an immune cell-engaging bispecific or multispecific antibody comprising the antigen-binding fragment (including an antigen-binding fragment of a humanized antibody) provided by the present invention and at least one scFv that binds to an immune cell-activating antigen.

In the present invention, a cytolytic synapse between cytotoxic T cells or NK cells and target cancer cells is temporarily induced by the immune cell-engaging bispecific or multispecific antibody to release toxic substances.

In the present invention, the T cell-activating antigen may be CD3, TCRα, TCRβ, TCRγ, TCRξ, HVEM, LIGHT, CD40, 4-1BB, OX40, DR3, GITR, CD30, TIM1, SLAM, CD2 or CD226, without being limited thereto.

In the present invention, the NK cell-activating antigen may be NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E or CD 160, without being limited thereto.

Other immune cell engagers are described in detail in U.S. Patent Publication No. 2017/0368169, which may be incorporated herein by reference.

Specifically, the immune cell-engaging bispecific or multispecific antibody may comprise a tandem scFv and is capable of binding to the following antigen and a surface antigen on cancer cells. The surface antigen on the cancer cells is mesothelin, which is targeted by the antibody according to the invention.

In the present invention, the immune cell-engaging bispecific or multispecific antibody may comprise, for example, a structure of a VL (mesothelin)-VH (mesothelin)-VH (CD3 or CD16A)-VL (CD3 or CD16A), VH (mesothelin)-VL (mesothelin)-VH (CD3 or CD16A)-VL (CD3 or CD16A), VH (CD3 or CD16A)-VL (CD3 or CD16A)-VH (mesothelin)-VL (mesothelin), or VH (CD3 or CD16A)-VL (CD3 or CD16A)-VL (mesothelin)-VH (mesothelin) form.

In the present invention, the immune cell-engaging bispecific or multispecific antibody may further comprise a linker between the heavy chain variable region and the light chain variable region. Here, the linker may be a peptide linker and may be about 10-25 aa in length. For example, the linker may include hydrophilic amino acids such as glycine and/or serine, without being limited thereto.

Specifically, the linker in the present invention may comprise, for example, (GS)n, (GGS)n, (GSGGS)n or (GnS)m (n and m are each an integer ranging from 1 to 10), preferably GGGGS, more preferably GGGGSGGGGSGGGGS represented by SEQ ID NO: 50, without being limited thereto.

In the present invention, examples of the immune cell-engaging bispecific or multi specific antibody include blinatumomab (Amgen), which binds to CD3 and CD19; solitomab (Amgen), which binds to CD3 and EpCAM; MEDI 565 (MedImmune, Amgen), which binds to CD3 and CEA; and BAY2010112 (Bayer, Amgen), which binds to CD3 and PSMA. Exemplary DARTs include MGD006 (Macrogenics), which binds to CD3 and CD123; and MGD007 (Macrogenics), which binds to CD3 and gpA33. Exemplary TandAbs include AFM11 (Affimed Therapeutics), which binds to CD3 and CD19; and AFM13 (Affimed Therapeutics), which binds to CD30 and CD16A.

### Immunoconjugate

According to another embodiment of the present invention, the present invention relates to an immunoconjugate in which the antibody (including humanized antibody) or antigen-binding fragment provided by the present invention is combined with a cytotoxic agent.

In the present invention, the cytotoxic agent may be at least one selected from the group consisting of a chemotherapeutic agent, a chemotherapeutic drug, a growth inhibitory agent, a toxin (e.g., a protein toxin, an enzymatically active toxin of bacterial, fungal, plant, or animal origin, or fragments thereof), or a radioactive isotope.

In one embodiment, the immunoconjugate provided by the present invention may be an antibody-drug conjugate (ADC) in which the antibody or antigen-binding fragment is conjugated to a drug.

As used herein, the term "antibody-drug conjugate (ADC)" refers to a form in which a drug and an antibody are chemically linked without degrading the biological activities of the antibody and the drug. In the present invention, the antibody-drug conjugate refers to a form in which the drug is conjugated to the N-terminal amino acid residue of the heavy and/or light chain of the antibody, specifically, a form in which the drug is conjugated to the N-terminal α-amine group of the heavy and/or light chain of the antibody.

In the present invention, the antibody or antigen-binding fragment may be conjugated to a drug via a linker. The linker is a site for linking the antibody to the drug, and is cleavable under intracellular conditions, that is, allows the drug to be released from the antibody in an intracellular environment. Also, the antibody is stable during systemic circulation by reflecting a long half-life of the antibody, and the linkage between the linker and the drug should not affect the stability and pharmacokinetics of the antibody.

In the present invention, the linker may include, for example, a cleavable linker or a non-cleavable linker. The cleavable linker such as a peptide linker may be cleaved by intracellular peptidase or protease such as lysosomal or endosomal protease, and the non-cleavable linker, for example, a thioether linker, allows the drug to be released after the antibody is non-selectively cleaved by intracellular hydrolysis.

In the present invention, the cleavable linker may include a peptide linker. The peptide linker is at least 2 amino acids in length. For example, a dipeptide of Val-Cit, Val-Ala, or Val-Cit or Phe-Leu or Gly-Phe-Leu-Gly may be included. Examples of the linker are described in detail in International Patent Publication No. WO2004/010957, which may be incorporated herein by reference.

In the present invention, the antibody-drug conjugate is internalized into cancer cells through an endosome-lysosome pathway after an antibody region of the ADC binds to the antigen of the target cancer cells to form an ADC-antigen complex. In this case, the intracellular release of the cytotoxic drug is regulated by the internal environment of the endosome/lysosome.

In the present invention, the cleavable linker is pH-sensitive and may be sensitive to hydrolysis at a specific pH value. In general, the pH sensitive linker may be hydrolyzed under acidic conditions. For example, acid labile linkers, which may be hydrolyzed in lysosomes, include hydrazone, semicarbazone, thiosemicarbazone, cis-aconitic amide, orthoester, acetal, ketal, and the like.

In the present invention, the linker may also be cleaved under reducing conditions, and for example, a disulfide linker may correspond thereto. Various disulfide bonds may be formed using N-succinimidyl-S-acetylthioacetate (SATA), N-succinimidyl-3-(2-pyridyldithio)propionate (SPDP), N-succinimidyl-3-(2-pyridyldithio)butyrate (SPDB), and N-succinimidyl-oxycarbonyl-alpha-methyl-alpha-(2-pyridyl-dithio)toluene (SMPT). These disulfide linkers may be cleaved by disulfide exchange with thiols of intracellular glutathione.

In the present invention, the drug and/or drug-linker may be randomly conjugated through lysines of the antibody or may be conjugated through cysteines exposed when the disulfide bond chain is reduced. In some cases, the linker-drug may be linked through cysteines present in a genetically engineered tag, for example, a peptide or protein. The genetically engineered tag, for example, a peptide or protein, may include, for example, an amino acid motif that may be recognized by isoprenoid transferase. The peptide or protein has deletion at the carboxyl terminus of the peptide or protein, or has addition by covalent bonding of a spacer unit to the carboxyl (C) terminus of the peptide or protein.

In the present invention, the peptide or protein may be covalently bonded directly to the amino acid motif or may be linked to the amino acid motif via covalent bonding to a spacer unit. The amino acid spacer unit consists of 1 to 20 amino acids, and is preferably a glycine unit.

In the present invention, the linker may include a beta-glucuronide linker that is recognized and hydrolyzed by β-glucuronidase, which is present in large numbers in lysosomes or overexpressed in some tumor cells. Unlike a peptide linker, the beta-glucuronide linker has the advantage of being capable of increasing the solubility of the antibody-drug conjugate when linked with a drug having high hydrophobicity due to its high hydrophilicity. In this regard, a beta-glucuronide linker disclosed in International Patent Publication No. WO2015/182984, for example, a beta-glucuronide linker containing a self-immolative group may be used, and the above document is incorporated herein by reference.

In the present invention, the linker may be, for example, a non-cleavable linker, and in this case, the drug is released through only one step of antibody hydrolysis in the cells to produce, for example, an amino acid-linker-drug complex. Such a type of linker may be a thioether group or a maleimidocaproyl group, and may maintain its stability in blood.

In the present invention, the linker-drug may be linked randomly through cysteines exposed when the disulfide bond chain of the antibody or antigen-binding fragment is reduced, or the linker-drug may be linked by introducing an antibody terminal binding peptide having the sequence GGGGGGGCVIM.

In the present invention, the drug may be an agent that exhibits pharmacological effects, and it may be conjugated to the antibody. Specifically, the drug may be a chemotherapeutic agent, a toxin, microRNA (miRNA), siRNA, shRNA, or a radioactive isotope. The chemotherapeutic agent may be, for example, a cytotoxic agent or an immunosuppressive agent. Specifically, the chemotherapeutic agent may include a microtubulin inhibitor, a mitotic inhibitor, a topoisomerase inhibitor, or a chemotherapeutic agent that may function as DNA intercalator. In addition, it may include a drug for treating cancer, inflammation, or immune disease. For example, the chemotherapeutic agent may include an immunomodulatory compound, an anticancer drug, an antiviral agent, an antibacterial agent, an antifungal agent, an anthelmintic agent, or a combination thereof.

In the present invention, the anticancer drug may be, for example, at least one selected from the group consisting of maytansinoid, auristatin (including MMAE and MMAF), aminopterin, actinomycin, bleomycin, thalisomycin, camptothecin, N8-acetyl spermidine, 1-(2 chloroethyl)-1,2-dimethyl sulfonyl hydrazide, esperamycin, etoposide, 6-mercaptopurine, dolastatin, trichothecene, calicheamicin, taxol, taxane, paclitaxel, docetaxel, methotrexate, vincristine, vinblastine, doxorubicin, melphalan, mitomycin A, mitomycin C, chlorambucil, duocamycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, CNU (bischloroethylnitrosourea), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, EICAR (5-ethynyl-1-beta-Dribofuranosylimidazole-4-carboxamide), hydroxyurea, deferoxamine, floxuridine, doxifluridine, raltitrexed, cytarabine (ara C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factor, gemcitabine, velcade, revamid, thalamid, lovastatin, 1-methyl-4-phenylpyridiniumion, staurosporine, actinomycin D, dactinomycin, bleomycin A2, bleomycin B2, peplomycin, epirubicin, pirarubicin, zorubicin, mitoxantrone, verapamil, thapsigargin, nuclease, and toxins derived from bacteria, animals, and plants, without being limited thereto.

In the present invention, examples of the anti-inflammatory agent include, but are not limited to, steroidal drugs that reduce inflammation or swelling by binding to glucocorticoid receptors, non-steroidal anti-inflammatory drugs (NSAIDs) that relieve pain by counteracting cyclic oxygenase (COX), which synthesizes prostaglandins that cause inflammation, or immune-specific anti-inflammatory drugs (ImSAIDs) that change the activation and transport of inflammatory cells.

In the present invention, the agent for treating immune disease include, but are not limited to, azathioprine, chlorambucil, cyclophosphamide, cyclosporine, mycophenolate, azathioprine, or methotrexate.

In the present invention, in some cases, the drug may include at least one nucleophilic group selected from the group consisting of amine, thiol, hydroxyl, hydrazide, oxime, hydrazine, thiosemicarbazone, hydrazine carboxylate, and arylhydrazide groups which may react to form covalent bonds with the linker and an electrophilic group on the linker reagent, without being limited thereto.

### Chimeric Antigen Receptor (CAR)

According to another embodiment of the present invention, the present invention relates to a chimeric antigen receptor (CAR) comprising an antigen-binding fragment (including a humanized antigen-binding fragment) provided by the present invention.

As used herein, the term "chimeric antigen receptor" or "CAR" refers to an engineered receptor comprising an extracellular antigen binding domain and an intracellular signaling domain. While the most common type of CAR comprises a single-chain variable fragment (scFv) derived from a monoclonal antibody fused to a transmembrane and intracellular domain of a T cell co-receptor, such as the CD3ζ chain, the present invention described herein is not limited to these domains. Rather, as used herein "chimeric antigen receptor" or "CAR" refers to any receptor engineered to express and extracellular antigen binding domain fused or linked to any intracellular signaling molecule.

In the present invention, the binding domain may include the antigen-binding fragment provided by the present invention, preferably an scFv.

The chimeric antigen receptor of the present invention may further include at least one of a hinge region (or spacer) or a signaling domain.

In the present invention, the hinge region is a portion that links the antigen-binding domain to the transmembrane domain and is also called "spacer". The hinge region has the purpose for expanding the antigen-binding domain from T cell membrane or NK cell membrane.

In the present invention, the hinge sequence may be obtained, for example, from any suitable sequence from any genus, including human or a part thereof, or may include a hinge region of a human protein including CD8, CD28, 4-1BB, OX40, all or part of CD3 zeta (ζ) chain, T cell receptor α or β chain, CD3ε, CD45, CD4, CD5, CD9, CD16, CD22, CD33, CD37, CD64, CD80, CD86, CD134, CD137, ICOS, CD154, functional derivatives thereof, or combinations thereof, which are commonly used in the art, without being limited thereto.

In addition, in the present invention, the hinge region may comprise one selected from, but not limited to, immunoglobulins (e.g. IgG1, IgG2, IgG3, IgG4, and IgD), without being limited thereto.

In the present invention, the hinge region may be a CD8 hinge region that comprises the amino acid sequence set forth in SEQ ID NO: 59 or is encoded by the nucleotide sequence set forth in SEQ ID NO: 60, without being limited thereto.

In the present invention, the signaling domain refers to the portion of the chimeric antigen receptor that is found or is engineered to be found inside a T cell. In the present invention, the signaling domain may or may not also contain a transmembrane domain which anchors the chimeric antigen receptor in the plasma membrane of a T cell.

In the present invention, the transmembrane domain and the signaling domain may be derived from the same protein (e.g., CD3ζ). Alternatively, the transmembrane domain and the signaling domain may be derived from different proteins (e.g., the transmembrane domain of CD28 and the intracellular signaling domain of a CD3ζ molecule, or vice versa).

In the present invention, the transmembrane domain comprises a hydrophobic polypeptide that spans the cellular membrane. In particular, the transmembrane domain may span from one side of a cell membrane (extracellular) through the other side of the cell membrane (intracellular or cytoplasmic).

In the present invention, the transmembrane domain may be in the form of an alpha helix or a beta barrel, or combinations thereof. In addition, in the present invention, the transmembrane domain may comprise a polytopic protein, which has many transmembrane segments, each alpha-helical, beta sheets, or combinations thereof.

In the present invention, the transmembrane domain may comprise, for example, a T cell receptor α or β chain, all or part of CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, without being limited thereto.

In the present invention, the transmembrane domain may be, for example, a polypeptide comprising hydrophobic residues such as leucine and valine, which is artificially designed. In one embodiment of the present invention, a triplet of phenylalanine, tryptophan and valine may be found at each end of the synthetic transmembrane domain.

In the present invention, the transmembrane domain may be a CD8 transmembrane domain that comprises the amino acid sequence set forth in SEQ ID NO: 61 or is encoded by the nucleotide sequence set forth in SEQ ID NO: 62, without being limited thereto.

In the present invention, at least one costimulatory domain may be further included between the transmembrane domain and the signaling domain. In the present invention, the costimulatory domain is a portion through which a costimulatory signal is transmitted. The costimulatory domain may serve to help CAR-T cells or CAR-NK cells that recognize a specific antigen bound to the antigen-binding domain to self-proliferate while eliciting an immune response, and serve to transmit a signal so as to increase the *in vivo* retention time of the cells.

In the present invention, the costimulatory domain may comprise a functional signaling domain derived from a polypeptide comprising a ligand that binds to 4-1BB (CD137), OX40, CD27, CD28, CD30, CD40, PD1, CD2, CD7, CD258, natural killing group 2 member C (NKG2C), natural killing group 2 member D (NKG2D), B7-H3, CD83, ICAM-1, LFA-1 (CD11a/CD18), or ICOS, active fragments thereof, functional derivatives thereof, or combinations thereof, without being limited thereto.

In the present invention, the costimulatory domain may be 4-1BB that comprises the amino acid sequence set forth in SEQ ID NO: 63 or is encoded by the nucleotide sequence set forth in SEQ ID NO: 64, without being limited thereto.

In the present invention, the signaling domain may comprise a polypeptide that provides activation of an immune cell to stimulate or activate at least some aspect of the immune cell signaling pathway.

In the present invention, the signaling domain may comprise a functional signaling domain derived from a polypeptide comprising all or part of CD3 zeta (ζ), common FcR gamma (FcER1G), Fc gamma RIIIa, FcR beta (Fc epsilon R1b), CD3 gamma, CD3 delta, CD3 epsilon, CD79a, CD79b, DNAX-activating protein 10 (DAP10), DNAX-activating protein 12 (DAP12), active fragments thereof, functional derivatives thereof, or combinations thereof, without being limited thereto. Such signaling domains are known in the art.

In the present invention, the signaling domain may be CD3 zeta (ζ) that comprises the amino acid sequence set forth in SEQ ID NO: 65 or is encoded by the nucleotide sequence set forth in SEQ ID NO: 66, without being limited thereto.

In addition, the chimeric antigen receptor of the present invention may further comprise a signal peptide.

In the present invention, the "signal peptide" may include a peptide sequence which is a peptide of any secreted or transmembrane protein that directs the transport of the chimeric antigen receptor of the present invention to the cell membrane and cell surface, and provides correct localization of the chimeric antigen receptor of the present invention. In particular, the signal peptide in the present invention directs the chimeric antigen receptor of the present invention to the cellular membrane, wherein the extracellular portion of the chimeric antigen receptor is displayed on the cell surface, the transmembrane portion spans the plasma membrane, and the active domain is in the cytoplasmic portion, or interior of the cell.

In the present invention, as the signal peptide, any peptide may be used without limitation as long as it has the above function. For example, the signal peptide may be an N-terminal CD8α signal peptide that comprises the amino acid sequence set forth in SEQ ID NO: 57 or is encoded by the nucleotide sequence set forth in SEQ ID NO: 58, without being limited thereto.

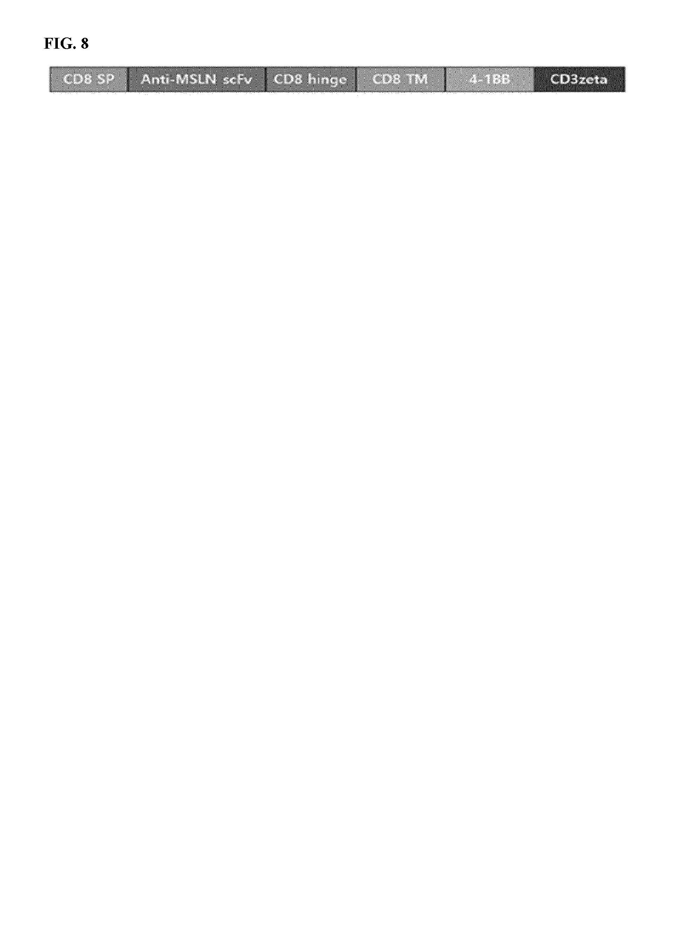

FIG. 8 schematically shows the structure of a chimeric antigen receptor according to one embodiment of the present invention. As shown therein, the chimeric antigen receptor may consist of or comprise a mesothelin binding domain (scFv (e.g., VL-linker-VH) according to the present invention-CD8 hinge-CD8 transmembrane domain-4-1BB-CD3ζ, or consist of or comprise a signal peptide-mesothelin binding domain (scFv according to the present invention)-CD8 hinge-CD8 transmembrane domain-4-1BB-CD3ζ. However, the structure of the chimeric antigen receptor of the present invention is not limited to the above structure.

As an example, the chimeric antigen receptor provided by the present invention may comprise an scFv of at least one of SEQ ID NOs: 51 or 53, and may comprise or consist of the amino acid sequence set forth in at least one of SEQ ID NOs: 68 or 70, without being limited thereto.

As an example, the chimeric antigen receptor provided by the present invention may comprise a humanized scFv of SEQ ID NO: 55, and may comprise or consist of the amino acid sequence set forth in SEQ ID NO: 72, without being limited thereto.

In addition, the chimeric antigen receptor provided by the present invention may comprise a sequence in which an N-terminal CD8α signal peptide comprising the amino acid sequence set forth in SEQ ID NO: 57 is further linked at the N-terminus of the amino acid sequence set forth in SEQ ID NO: 68 70, or 72. Specifically, the chimeric antigen receptor may comprise or consist of the amino acid sequence set forth in SEQ ID NO: 74, 76, or 78, without being limited thereto.

According to another embodiment of the present invention, the present invention relates to a nucleic acid molecule encoding the chimeric antigen receptor according to the present invention.

In the present invention, the nucleic acid molecule encoding the chimeric antigen receptor is easily produced from the amino acid sequence of the specified chimeric antigen receptor by any conventional method. A nucleotide sequence encoding the amino acid sequence can be obtained from the aforementioned NCBI RefSeq IDs or accession numbers of GenBenk for the amino acid sequence of each domain, and the nucleic acid of the present invention can be produced using a standard molecular biological and/or chemical procedure. For example, based on the nucleotide sequence, the polynucleotide can be synthesized, and the nucleic acid molecule of the present invention can be produced by combining DNA fragments obtained from a cDNA library using a polymerase chain reaction (PCR).

The nucleic acid molecule of the present invention may be part of a gene or an expression or cloning cassette, without being limited thereto.

As an example, the nucleic acid molecule of the present invention may comprise or consist of the nucleotide sequence set forth in SEQ ID NO: 69, which encodes a chimeric antigen receptor comprising the amino acid sequence set forth in SEQ ID NO: 68, without being limited thereto.

As an example, the nucleic acid molecule of the present invention may comprise or consist of the nucleotide sequence set forth in SEQ ID NO: 71, which encodes a chimeric antigen receptor comprising the amino acid sequence set forth in SEQ ID NO: 70, without being limited thereto.

As an example, the nucleic acid molecule of the present invention may comprise or consist of the nucleotide sequence set forth in SEQ ID NO: 73, which encodes a chimeric antigen receptor comprising the amino acid sequence set forth in SEQ ID NO: 72, without being limited thereto.

According to another embodiment of the present invention, the present invention relates to an expression cassette comprising the nucleic acid molecule provided by the present invention.

The expression cassette of the present invention may contain expression regulatory sequences capable of regulating expression of the nucleic acid molecule of the present invention, such as a promoter, an enhancer, a polyadenylation signal, a transcription terminator or an internal ribosome entry site (IRES).

In the present invention, examples of the promoter include, but are not limited to, an SFFV promoter, an elongation factor 1a (EF 1a) promoter, or a CAG promoter (chicken beta-actin promoter with a CMV enhancer).

In the present invention, the promoter may be an EFla promoter comprising the nucleotide sequence set forth in SEQ ID NO: 67, without being limited thereto.

The expression cassette of the present invention may further comprise a truncated EGFR gene (EGFRt) as an additional gene sequence useful for monitoring expression thereof. In the present invention, the EGFRt is a non-immunogenic selection tool. For example, the EGFRt may be used to select T cells or NK cells transformed with an EGFRt-containing cassette in immunomagnetic selection performed using biotinylated mesothelin and anti-biotin microbeads, and may also be used as a tracking marker in flow cytometry for T cell or NK cell tracking. In addition, the EGFRt may be used as a suicide gene through the mesothelin-mediated antibody dependent cellular cytotoxicity (ADCC) pathway.

In addition, in the present invention, the truncated EGFR gene (EGFRt) and one end of the Fc γ receptor binding domain of the polynucleotide of the present invention may be linked together by a ribosomal skip element which is a cleavable linker.

As used herein, the term "ribosomal skip" refers to an alternative mechanism of translation in which a specific peptide prevents the ribosome of a cell from covalently linking a new inserted amino acid and instead allows it to continue translation thus resulting in a co-translational cleavage of the polyprotein. This process is induced by a "2A ribosomal skip" element or cis-acting hydrolase element (e.g., CHYSEL sequence). In some embodiments, this sequence comprises a non-conserved amino acid sequence with a strong alpha-helical propensity followed by the consensus sequence -D(V/I)ExNPG P, where x is any amino acid. Apparent cleavage occurs between G and P. In the present invention, the ribosomal skip element may be a 2A ribosomal skip element, preferably a 5' T2A ribosomal skip element, without being limited thereto.

According to another embodiment of the present invention, the present invention relates to a recombinant expression vector comprising the expression cassette provided by the present invention.

In the present invention, details regarding the recombinant expression vector overlap with those described above with respect to the antibody or antigen-binding fragment, and thus the description thereof will be omitted below.

According to another embodiment of the present invention, the present invention relates to an immune effector cell transduced with the recombinant expression vector provided by the present invention.

As used herein, the term "immune effector cell" may be a lymphocyte that participates in an immune response, such as promoting an immune effector response.

As used herein, the term "lymphocytes" refers to cells that are commonly found in lymph and include natural killer cells (NK cells), T cells, and B cells. Those skilled in the art will understand that the immune cell types listed above can be further divided into subtypes.

As used herein, the term "natural killer cells (NK cells)" is defined as large granular lymphocytes (LGLs), which constitute three types of cells differentiated from the common lymphoid progenitor-generating B and T lymphocytes. NK cells are known to differentiate and mature in the bone marrow, lymph nodes, spleen, tonsils, and thymus, where they then enter into the circulation. In the present invention, the NK cells include, without limitation, any type of NK cells, for example, cultured NK cells, such as primary NK cells, NK cells from a cultured NK cell line, or NK cells obtained from a mammal, without being limited thereto. If obtained from a mammal, the NK cell can be obtained from numerous sources, including but not limited to blood, bone marrow, lymph node, the thymus, or other tissues or fluids. NK cells can also be enriched for or purified. The NK cells are preferably human NK cells (e.g., isolated from a human). NK cell lines are available from, e.g., the American Type Culture Collection (ATCC), and include, for example, NK-92 cells (ATCC CRL-2407), NK92MI cells (ATCC CRL-2408), and derivatives thereof.

According to another embodiment of the present invention, the present invention relates to a polypeptide expressed from the immune effector cell provided by the present invention.

As an example, the polypeptide expressed from the immune effector cell provided by the present invention may comprise or consist of the amino acid sequence set forth in any one of SEQ ID NOs: 68 and 70, without being limited thereto.

As an example, the polypeptide expressed from the immune effector cell provided by the present invention may comprise or consist of the amino acid sequence set forth in SEQ ID NO: 72, without being limited thereto.

### 3. Therapeutic Use

According to another embodiment of the present invention, the present invention relates to a pharmaceutical composition for preventing, ameliorating or treating mesothelin-related disease, comprising, as an active ingredient, the antibody (including humanized antibody) or antigen-binding fragment provided by the present invention, the chimeric antigen receptor provided by the present invention, or an immune effector cell expressing the chimeric antigen receptor.

In the present invention, the mesothelin-related disease may be a disease mediated by mesothelin overexpression, for example, cancer or a tumor.

As used herein, the term "cancer" or "tumor" refers to or describes the physiological condition in mammals that is typically characterized by unregulated cell growth. In the present invention, the cancer to be prevented, ameliorated or treated may be a cancer or tumor in which mesothelin is overexpressed. Although the cancer is not particularly limited, it may be, for example, liver cancer, lung cancer, pancreatic cancer, non-small cell lung cancer, colon cancer, bone cancer, skin cancer, head or neck cancer, skin or intraocular melanoma, uterine cancer, ovarian cancer, rectal cancer, gastric cancer, perianal cancer, rectal cancer, breast cancer, fallopian tube carcinoma, endometrial carcinoma, cervical carcinoma, vaginal carcinoma, vulvar carcinoma, Hodgkin's disease, esophageal cancer, small intestine cancer, endocrine adenocarcinoma, thyroid cancer, parathyroid cancer, adrenal cancer, soft tissue sarcoma, urethral cancer, penile cancer, prostate cancer, chronic or acute leukemia, lymphocytic lymphoma, bladder cancer, renal or ureteral cancer, renal cell carcinoma, renal pelvic carcinoma, central nervous system (CNS) tumor, primary central nervous system lymphoma, spinal cord tumor, brainstem glioma, or pituitary adenoma, without being limited thereto.

In the present invention, the term "prevention" may include, without limitation, any action that can block symptoms caused by mesothelin-related disease, or suppress or delay the symptoms, by using the composition of the present invention.

In the present invention, the terms "amelioration" and "treatment" may include, without limitation, any action that can alleviate or beneficially change the symptoms caused by mesothelin-related disease, by using the composition of the present invention.

For use, the pharmaceutical composition of the present invention may be formulated in the form of, but not limited to, oral preparations, such as powders, granules, capsules, tablets, and aqueous suspensions, as well as external preparations, suppositories, and sterile injectable solutions, according to the respective conventional methods. Preferably, the pharmaceutical composition may be formulated for intratracheal administration or administration by inhalation or as an injection, without being limited thereto.

The pharmaceutical composition of the present invention may contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preservative, and the like may be used for topical administration. In addition, the pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative, or the like.

The routes of administration of the pharmaceutical composition according to the present invention include, but are not limited to, oral, intravenous, intramuscular, intraarterial, intramedullary, intradural, intracardiac, transdermal, subcutaneous, intraperitoneal, intranasal, gastrointestinal, topical, sublingual and intrarectal routes. The pharmaceutical composition may be administered orally or parenterally, preferably parentally, without being limited thereto.

In the present invention, the "parenteral" includes subcutaneous, transdermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

The pharmaceutical composition of the present invention may vary depending on various factors, including the activity of a particular compound used, the patient's age, body weight, general health, sex and diet, administration time, the route of administration, excretion rate, drug combination, and the severity of a particular disease to be prevented or treated. Although the dose of the pharmaceutical composition varies depending on the patient's condition, body weight, the severity of the disease, the form of drug, the route of administration, and the duration of administration, it may be appropriately selected by a person skilled in the art. The pharmaceutical composition may be administered at a dose of 0.0001 to 50 mg/kg/day or 0.001 to 50 mg/kg/day. The pharmaceutical composition may be administered once or several times a day. The dose does not limit the scope of the present invention in any way. The pharmaceutical composition according to the present invention may be formulated as pills, sugar-coated tablets, capsules, liquids, gels, syrups, slurries, or suspensions.

According to another embodiment of the present invention, the present invention relates to a method for preventing, ameliorating or treating mesothelin-related disease, comprising a step of administering a therapeutically effective amount of the composition to a subject in need of administration.

In the present invention, the subject is a subject having or suspected of having a mesothelin-related disease (particularly, a cancer or tumor in which mesothelin is overexpressed). The subject suspected of having the disease refers to all animals, including humans, monkeys, cows, horses, sheep, pigs, chickens, turkeys, quails, cats, dogs, mice, rats, rabbits, or guinea pigs, that have or may develop the disease, but includes, without limitation, subjects that can be treated with the active ingredient provided by the present invention.

As used herein, the term "pharmaceutically effective amount" is an amount sufficient to stop or alleviate the physiological effects caused by mesothelin-related disease symptoms in a subject or patient. The appropriate effective amount can be decided by the attending physician within the scope of sound medical judgment, and may be administered once or in divided doses. However, for the purposes of the present invention, the specific therapeutically effective amount for a particular patient is preferably applied differently depending on various factors, including the type and degree of response to be achieved, the specific composition containing the active ingredient, including whether other agents are used as the case may be, the patient's age, body weight, general health status, sex, and diet, the time of administration, the route of administration, the frequency of administration, the secretion rate of the composition comprising the active ingredient, the duration of treatment, and drugs used simultaneously or in combination with the specific composition, and similar factors well known in the medical field.

The total effective amount of the composition of the present invention may be administered to a patient as a single dose, or may be administered using a fractionated treatment protocol in which multiple doses are administered over a more prolonged period of time. The composition of the present invention may have varying contents of the active ingredient depending on the severity of the disease. Specifically, a preferred total dose of the composition of the present invention may be about 0.0001 mg to 500 mg per kg of patient body weight per day. However, the dose of the composition for a patient is determined in view of various factors such as the patient's age, body weight, health status, sex, severity of the disease, diet, and excretion rate, as well as the route of administration and number of administrations of the pharmaceutical composition. Considering these factors, a person skilled in the art will be able to determine an appropriate effective dose according to the specific use of the composition of the present invention. The pharmaceutical composition according to the present invention is not particularly limited in its formulation, administration route, and administration mode, as long as it exhibits the effects of the present invention.

In addition, in the present invention, the method for preventing, ameliorating or treating mesothelin-related disease may be a combination therapy that further comprises administering a compound or substance having therapeutic activity against one or more diseases.

In the present invention, the "combination" should be understood to refer to simultaneous, individual, or sequential administration. When the administration is done in a sequential or individual manner, the second component should be administered at intervals such that beneficial effects of the combination are not lost.

### 4. Diagnostic Use

According to another embodiment of the present invention, the present invention relates to a composition for diagnosing mesothelin-related disease comprising the antibody (including humanized antibody) or antigen-binding fragment provided by the present invention, or the bispecific or multispecific antibody.

Mesothelin, which is detected by the antibody or antigen-binding fragment of the present invention, is known to be expressed at high levels in cancer or tumor cells. Therefore, in the present invention, mesothelin may be detected using the antibody or antigen-binding fragment and used as a diagnostic marker for diagnosis of cancer or tumor, and evaluation of disease progression and prognosis before and after treatment.

The antibody or antigen-binding fragment of the invention may be used in any known assay methods, such as competitive binding assays, direct and indirect sandwich assays, and immunoprecipitation assays.

The antibody or antigen-binding fragment of the invention may be provided with a label that functions to: (i) provide a detectable signal; (ii) interact with a second label to modify the detectable signal provided by the first or second label, e.g. FRET (Fluorescence Resonance Energy Transfer); (iii) affect mobility, e.g. electrophoretic mobility, by charge, hydrophobicity, shape, or other physical parameters, or (iv) provide a capture moiety, e.g., affinity, antibody/antigen, or ionic complexation. Suitable as label are fluorescent labels, luminescent labels, chromophore labels, radioisotopic labels, isotopic labels, isobaric labels, enzyme labels, particle labels, in particular metal particle labels, magnetic particle labels, polymer particle labels, small organic molecules such as biotin, ligands of receptors or binding molecules such as cell adhesion proteins or lectins, label-sequences comprising nucleic acids and/or amino acid residues which can be detected by use of binding agents, etc.

In the present invention, the specific type of the label is not particularly limited, but examples of such labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (see U.S. Pat. No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

According to another embodiment of the present invention, the present invention relates to a kit for diagnosing mesothelin-related disease comprising the diagnostic composition of the present invention.

In the present invention, the term "kit" refers to a tool in which a probe or antibody that binds specifically to a biomarker component is labeled with a detectable substance so that the expression level of the biomarker may be assessed. The term "labeled", with regard to the probe or antibody, is intended to encompass direct labeling of the probe or antibody by the reaction of a detectable substance with a substrate, as well as indirect labeling of the probe or antibody by reactivity with another reagent that is directly labeled. The kit may comprise a chromogenic substrate solution to induce a chromogenic reaction with the label, a washing liquid, and other solutions, and may be prepared to comprise reagent components used. In the present invention, the kit may be a kit comprising essential components necessary for performing RT-PCR, and may comprise, in addition to each primer pair specific for the marker gene, a test tube, a reaction buffer, deoxynucleotides (dNTPs), Taq-polymerase, reverse transcriptase, DNase and RNase inhibitors, sterile water, and the like. The kit may also be a kit for detecting a gene for cancer diagnosis, comprising essential components necessary for performing DNA chip assay. The DNA chip kit may comprise a substrate to which a cDNA corresponding to a gene or a fragment thereof is attached as a probe, wherein the substrate may comprise a cDNA corresponding to a quantitative control gene or a fragment thereof. The kit of the present invention is not limited thereto and may be any type of kit known in the art.

In the present invention, the kit may be an RT-PCR kit, a DNA chip kit, an ELISA kit, a protein chip kit, a rapid kit, or a multiple reaction monitoring (MRM) kit.

The kit of the present invention may further comprise a composition, a solution or a device, which contains one or more different components suitable for the analysis method. For example, the kit in the present invention may further comprise essential components required for performing reverse transcription polymerase reaction. The reverse transcription polymerase reaction kit comprises a primer pair specific to the gene encoding the marker protein. The primer is an oligonucleotide having a sequence specific to the nucleotide sequence of the gene and may be about 7 to 50 bp in length, preferably about 10 to 30 bp in length. In addition, the kit may comprise a primer specific to the nucleotide sequence of a control gene. In addition, the reverse transcription polymerase reaction kit may comprise a test tube or other appropriate container, a reaction buffer (various pHs and magnesium concentrations), deoxynucleotides (dNTPs), enzymes such as Taq-polymerase and reverse transcriptase, DNAse and RNAse inhibitors, DEPC-water, sterilized water, etc.

In addition, the kit for diagnosing according to the present invention may comprise essential components required for performing DNA chip assay. The DNA chip kit may comprise a substrate having immobilized thereon a cDNA or oligonucleotide corresponding to each gene or a fragment thereof, a reagent for constructing a fluorescence-labeled probe, an agent, an enzyme, and the like. In addition, the substrate may comprise a cDNA or oligonucleotide corresponding to a control gene or a fragment thereof.

In addition, the kit for diagnosing according to the present invention may comprise essential components required for performing ELISA. The ELISA kit comprises an antibody specific to the protein. The antibody is a monoclonal antibody, a polyclonal antibody or a recombinant antibody, which has a high specificity and affinity for the marker protein and shows little or no cross-reactivity with other proteins. Also, the ELISA kit may comprise an antibody specific to a control protein. In addition, the ELISA kit may comprise reagents capable of detecting bound antibodies, for example, labelled secondary antibodies, chromophores, enzymes (e.g., conjugated with antibodies) and the substrates thereof or other substances which are capable of binding to antibodies.

In the present invention, a support for the antigen-antibody binding reaction may be selected from among nitrocellulose membranes, PVDF membranes, well plates made of polyvinyl or polystyrene resin, and slide glasses, without being limited thereto.

In the present invention, the washing solution preferably contains a phosphate buffer solution, NaCl, and Tween 20, and is more preferably a buffer solution (PBST) composed of a 0.02 M phosphate buffer solution, 0.13 M NaCl, and 0.05% Tween 20. After the secondary antibody is allowed to react with the antigen-antibody conjugate after the antigen-antibody binding reaction, a proper amount of the washing solution is added to the support to wash the support 3 to 6 times. A sulfuric acid (H₂SO₄) may be preferably used as the reaction stop solution.

According to another embodiment of the present invention, the present invention relates to a method of providing information for diagnosis of mesothelin-related disease, the method comprising a step of: bringing the antibody (including humanized antibody) or antigen-binding fragment provided by the present invention, or a bispecific or multispecific antibody comprising the antibody or antigen-binding fragment, into contact with a biological sample isolated from a subject of interest.

In the present invention, the subject is a subject having or suspected of having a mesothelin-related disease (particularly, a cancer or tumor caused by mesothelin overexpression). The subject suspected of having the disease refers to all animals, including humans, monkeys, cows, horses, sheep, pigs, chickens, turkeys, quails, cats, dogs, mice, rats, rabbits, or guinea pigs, that have or may develop the disease, but includes, without limitation, any subject in whom/which the disease can be diagnosed with the active ingredient provided by the present invention.

In the present invention, the biological sample may comprise whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid, without being limited thereto.

The method of providing information according to the present invention may comprise a step of measuring the expression level of mesothelin protein in the biological sample using the antibody or antigen-binding fragment provided by the present invention.

In the present invention, measurement of the expression level of the protein may be performed by Western blotting, ELISA (enzyme linked immunosorbent assay), competitive binding assay, or similar methods.

The method of providing information according to the present invention may comprise a step of predicting that the mesothelin-related disease has occurred or is highly likely to develop, when the expression level of mesothelin protein measured in the biological sample is higher than a control. Here, the control may be the expression level of mesothelin in a healthy normal control group, without being limited thereto.

### Advantageous Effects

The anti-mesothelin antibody or antigen-binding fragment of the present invention is able to bind to mesothelin with high affinity and avidity (binding capacity).

In addition, the humanized antibody or antigen-binding fragment provided by the present invention not only has high affinity and avidity to mesothelin antigen, but also has low immunogenicity even when administered to the human body.

Accordingly, the antibody or antigen-binding fragment provided by the present invention may be usefully applied to the treatment or diagnosis of mesothelin-mediated disease, such as cancer or a tumor, and may also be used to develop various antibody-drug conjugates, bispecific antibodies, or chimeric antigen receptors (CARs).

### Brief Description of Drawings

FIG. 1 shows the results of analyzing by ELISA the change in antibody titer for each mouse depending on the number of weeks after mouse immunization in Example 1.
FIG. 2 shows the results of yeast display antigen affinity selection in Example 1.
FIG. 3 shows the results of yeast display antigen affinity biopanning in Example 1.
FIG. 4 shows the results of analyzing by FACS the binding capacity of mesothelin-specific clones #19 and #45 selected in Example 1 to mesothelin.
FIG. 5 shows the results of purifying scFv from antibody clone #19 and antibody clone #45 screened in Example 1.
FIG. 6 shows the results of analyzing by ELISA the binding capacity of the mesothelin-specific scFvs (#19 and #45) according to the present invention to mesothelin antigen in Example 2.
FIG. 7 shows the results of analyzing by FACS the binding capacity of the mesothelin-specific scFvs (#19 and #45) according to the present invention to mesothelin-expressing K562 cells in Example 2.
FIG. 8 shows the structure of a mesothelin-specific chimeric antigen receptor construct according to an embodiment of the present invention.
FIG. 9 shows the results obtained in Example 3 by transfecting cells to express the mesothelin-specific CAR according to the present invention, and then selecting cells capable of binding to mesothelin antigen, and then analyzing by FACS whether the cells express the mesothelin-specific CAR.
FIG. 10 shows the results obtained in Example 3 by analyzing by FACS whether CD56, CD16 and CAR are expressed in selected cells transfected to express the mesothelin-specific CAR according to the present invention.
FIG. 11 shows the results of analyzing the death rate of cancer cells by FACS after co-culturing mesothelin-expressing pancreatic cancer cells (Capan-2) and mesothelin-specific CAR-expressing NK-92 cells according to the present invention in Example 4.
FIG. 12 shows the results of analyzing by SPR method the binding affinity of the mesothelin-specific humanized antibody (#19-1) according to the present invention to mesothelin antigen in Example 7.
FIG. 13 shows a map of a vector comprising a polynucleotide encoding a mesothelin-targeting chimeric antigen receptor according to the present invention in Example 8.
FIG. 14 shows the results of analyzing by FACS the binding capacity of 293T cells, transfected to express the mesothelin-specific CAR according to the present invention, to mesothelin in Example 8.
FIG. 15 shows the results of analyzing the mean fluorescence intensity (MFI) of cell staining as a measure of the binding of 293T cells, transfected to express the mesothelin-specific CAR according to the present invention, to mesothelin in Example 8.
FIG. 16 shows the results of analyzing by FACS whether the mesothelin-specific CAR according to the present invention is expressed in NK cells transfected to express the CAR, in Example 9.
FIG. 17 shows the results of analyzing by FACS whether CD3, CD56 and CD 16 are expressed in NK cells transfected to express the mesothelin-specific CAR according to the present invention, in Example 9.
FIG. 18 shows the results of analyzing the death rate of cancer cells by FACS after co-culturing mesothelin-expressing pancreatic cancer cells (Capan-2) and the mesothelin-specific CAR-expressing NK cells according to the present invention in Example 10.
FIG. 19 shows the results of analyzing the death rate of cancer cells by FACS after co-culturing mesothelin-expressing liver cancer cells (HepG2) and the mesothelin-specific CAR-expressing NK cells according to the present invention in Example 10.
FIG. 20 shows the results of analyzing the death rate of cancer cells by FACS after co-culturing mesothelin-expressing breast cancer cells (MDA-MB-231) and the mesothelin-specific CAR-expressing NK cells according to the present invention in Example 10.

### Best Mode

According to one embodiment of the present invention, the present invention relates to an antibody or antigen-binding fragment thereof that binds specifically to mesothelin (MSLN).

The mesothelin-binding antibody or antigen-binding fragment of the present invention may comprise a heavy chain variable region comprising a heavy chain CDR1 comprising the amino acid sequence of at least one of SEQ ID NOs: 3 or 17; a heavy chain CDR2 comprising the amino acid sequence of at least one of SEQ ID NOs: 5 or 19; and a heavy chain CDR3 comprising the amino acid sequence of at least one of SEQ ID NOs: 7 or 21, and comprise a light chain variable region comprising a light chain CDR1 comprising the amino acid sequence of SEQ ID NO: 10; a light chain CDR2 comprising the amino acid sequence of SEQ ID NO: 12; and a light chain CDR3 comprising the amino acid sequence of at least one of SEQ ID NOs: 14 or 26.

The mesothelin-binding antibody or antigen-binding fragment of the present invention may comprise at least one of the following antibodies or antigen-binding fragments:
1) an antibody or antigen-binding fragment comprising a heavy chain variable region comprising a heavy chain CDR1 comprising the amino acid sequence set forth in SEQ ID NO: 3, a heavy chain CDR2 comprising the amino acid sequence set forth in SEQ ID NO: 5, and a heavy chain CDR3 comprising the amino acid sequence set forth in SEQ ID NO: 7; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 14; and
2) an antibody or antigen-binding fragment comprising a heavy chain variable region comprising a heavy chain CDR1 comprising the amino acid sequence set forth in SEQ ID NO: 17, a heavy chain CDR2 comprising the amino acid sequence set forth in SEQ ID NO: 19, and a heavy chain CDR3 comprising the amino acid sequence set forth in SEQ ID NO: 21; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 26.

The mesothelin-binding antibody or antigen-binding fragment of the present invention may comprise a heavy chain variable region comprising the amino acid sequence of at least one of SEQ ID NOs: 38 or 42; and a light chain variable region comprising the amino acid sequence of at least one of SEQ ID NOs: 39 or 43.

The mesothelin-binding antibody or antigen-binding fragment of the present invention may comprise at least one of the following antibodies or antigen-binding fragments:
1) an antibody or antigen-binding fragment comprising a heavy chain variable region comprising the amino acid sequence set forth in SEQ ID NO: 38, and a light chain variable region comprising the amino acid sequence set forth in SEQ ID NO: 39; and
2) an antibody or antigen-binding fragment comprising a heavy chain variable region comprising the amino acid sequence set forth in SEQ ID NO: 42, and a light chain variable region comprising the amino acid sequence set forth in SEQ ID NO: 43.

In addition, the mesothelin-binding antigen-binding fragment of the present invention may be an scFv and may comprise the amino acid sequence of at least one of SEQ ID NOs: 51 or 53.

The present invention provides a mesothelin-binding humanized antibody or an antigen-binding fragment thereof. The mesothelin-binding humanized antibody or antigen-binding fragment thereof may comprise a heavy chain variable region comprising a heavy chain CDR1 comprising the amino acid sequence set forth in SEQ ID NO: 29; a heavy chain CDR2 comprising the amino acid sequence set forth in SEQ ID NO: 5; and a heavy chain CDR3 comprising the amino acid sequence set forth in SEQ ID NO: 32.

The mesothelin-binding humanized antibody or antigen-binding fragment thereof according to the present invention may comprise a heavy chain variable region comprising the amino acid sequence set forth in SEQ ID NO: 46; and a light chain variable region comprising the amino acid sequence set forth in SEQ ID NO: 47.

In addition, the mesothelin-binding humanized antigen-binding fragment of the present invention may be an scFv comprising the amino acid sequence of SEQ ID NO: 55.

According to another embodiment of the present invention, the present invention relates to a nucleic acid molecule encoding the antibody (including humanized antibody) or antigen-binding fragment provided by the present invention, a recombinant expression vector comprising the same, or a host cell transfected with the expression vector.

According to another embodiment of the present invention, the present invention relates to a bispecific or multispecific antibody or immunoconjugate comprising the antibody (including humanized antibody) or antigen-binding fragment provided by the present invention.

According to another embodiment of the present invention, the present invention relates to a chimeric antigen receptor (CAR) comprising the antigen-binding fragment (including humanized antigen-binding fragment) provided by the present invention.

The chimeric antigen receptor of the present invention may further comprise at least one of a hinge region (or a spacer) or a signaling domain.

The chimeric antigen receptor provided by the present invention may comprise an scFv of at least one of SEQ ID NOs: 51 or 53, and may comprise or consist of the amino acid sequence set forth in at least one of SEQ ID NOs: 68 or 70, without being not limited thereto.

In addition, the chimeric antigen receptor provided by the present invention may comprise a humanized scFv of SEQ ID NO: 55, and may comprise or consist of the amino acid sequence set forth in SEQ ID NOs: 72, without being limited thereto.

According to another embodiment of the present invention, the present invention relates to a nucleic acid molecule encoding the chimeric antigen receptor according to the present invention, an expression cassette comprising the nucleic acid molecule, a recombinant expression vector comprising the expression cassette, or an immune effector cell transduced with expression vectors.

According to another embodiment of the present invention, the present invention relates to the use of the antibody (including humanized antibody) or antigen-binding fragment provided by the present invention, the chimeric antigen receptor provided by the present invention, or an immune effector cells expressing the chimeric antigen receptor, for the prevention, amelioration or treatment of mesothelin-related disease.

In the present invention, the mesothelin-related disease may be a disease mediated by mesothelin overexpression, for example, cancer or a tumor.

According to another embodiment of the present invention, the present invention relates to the use of the antibody (including humanized antibody) or antigen-binding fragment provided by the present invention, or the bispecific or multispecific antibody, for detection of mesothelin or diagnosis of mesothelin-related disease.

### Mode for Invention

Hereinafter, the present invention will be described in more detail by way of examples. These examples are intended merely to illustrate the present invention in more detail, and it will be apparent to those skilled in the art that the scope of the present invention according to the subject matter of the present invention is not limited by these examples.

### EXAMPLES

### [Example 11 Screening and Binding Capacity Evaluation of Mesothelin-Specific

### Mouse Antibodies

### 1. Mouse Immunization

In order to obtain immunized mice required for the development of mesothelin-specific antibodies, the recombinant human mesothelin protein antigen set forth in SEQ ID NO: 1 and Imject^{™} Alum Adjuvant (Thermo Scientific^{™}) were mixed together and injected intraperitoneally into 6-week-old male Balb/c mice (n=4). Specifically, an emulsion was prepared by mixing 600 µL of the antigen prepared at a concentration of 0.5 µg/µL and 600 µL of alum adjuvant at a ratio of 1:1, and the emulsion was injected intraperitoneally into the mice at a final antigen concentration of 200 µL/dose/time. Immunization was performed a total of three times at two-week intervals using the same method as the first immunization method.

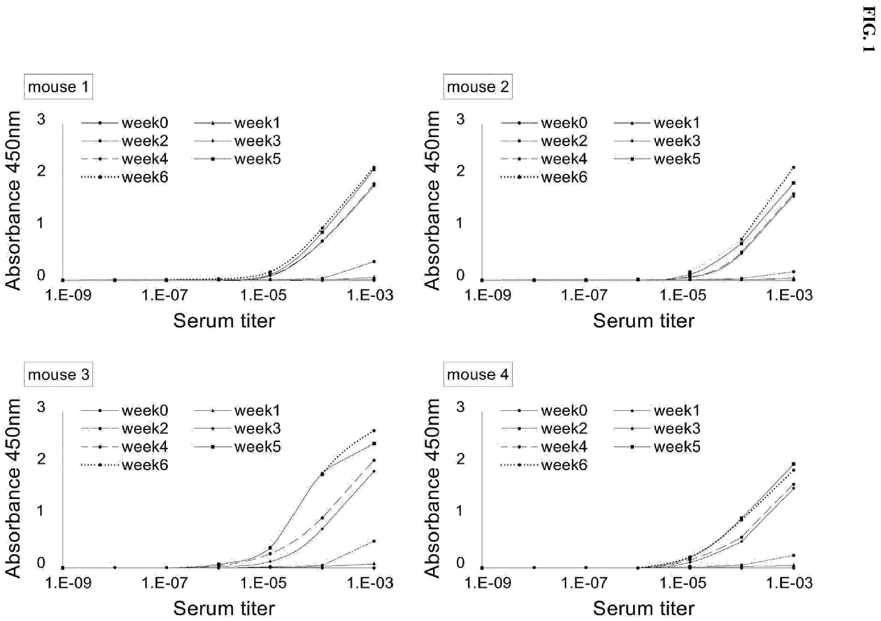

In order to check whether the mice were immunized, blood was collected from the tail of each mouse every week to obtain serum, and then whether antibody titer against the immunogen increased was examined by ELISA. Specifically, each well of an ELISA 96-well plate was coated with mesothelin mature form-his antigen overnight at 4°C, and then 3% BSA-containing PBS was added thereto, followed by incubation for 1 hour at room temperature. Mouse serum was serially diluted 1/10 from a dilution ratio of 10⁻³ and then incubated at room temperature for 1 hour. After washing three times with PBST (0.05% tween20), the plate was treated with anti-mouse IgG HRP (diluted 20,000:1) and incubated at room temperature for 1 hour. After washing three times with PBST (0.05% tween20), the plate was treated with TMB and incubated at room temperature for 20 minutes. After treatment with 2M H₂SO₄, the absorbance at 450 nm was measured. As a result, as shown in FIG. 1, it could be seen that antibody titer increased every week in all of the four mice, indicating that immunization was sufficiently induced.

### 2. Synthesis of cDNA

After euthanizing the mice immunized as described above, the spleen and bone marrow were harvested to obtain spleen cells and bone marrow cells, and then total RNA was extracted from the cells. For immune antibody library PCR, cDNA was synthesized using the extracted RNA as a template using SuperScript^{™} III First-Strand Synthesis SuperMix (Invitrogen).

### 3. Construction of E. coli Antibody Library

1 µL of the above-synthesized cDNA, 1 µL of forward primer mixture, 1 µL of reverse primer mixture, 4 µL of dNTP mixture, 10 µL of 5x Phusion buffer, and 0.5 µL of Phusion polymerase were added and the total volume was adjusted to 50 µL with water. Next, VH and Vk genes were obtained by performing PCR under the following conditions: 98°C for 3 min, 10 cycles, each consisting of 98°C for 30 sec, 55°C for 30 sec, and 72°C for 40 sec, and then 25 cycles, each consisting of 98°C for 30 sec, 60°C for 30 sec, and 72°C for 40 sec, followed by 72°C for 3 min.

Using the pYDS-H sfiI vector and the pYDS-K sfiI vector as templates, CH1 and CL genes were obtained by performing PCR under the following conditions: 98°C for 3 min, and then 35 cycles, each consisting of 98°C for 30 sec, 55°C for 30 sec, and 72°C for 30 sec, followed by 72°C for 3min.

The VH, Vk, CH1, and CL genes obtained by performing PCR were visualized on 1% agarose gel to confirm the DNA bands, and then subjected to gel extraction. VH and Vk were subjected to overlapping PCR with CH1 and CL, respectively. Here, the PCR was performed under the same conditions as described above. For reference, primer sequence information used in the above experiment is shown in Tables 8 to 14 below. However, in the present specification, in the primer sequences, 'R' may denote A or G, 'Y' may denote C or T, 'S' may denote G or C, 'W' may denote A or T, 'K' may denote G or T, 'M' may denote A or C, and 'D' may denote A or G or T.

pYDS-H-SfiI, pYDS-K-SfiI, VH-CHI (HC), and Vk-CL (LC) were each treated with sfiI (NEB), visualized on 1% agarose gel to confirm the bands, and then subjected to gel extraction to obtain DNA. Next, using T4 DNA ligase (NEB), the pYDS-H-SfiI vector and HC were ligated together, and the pYDS-k-SfiI vector and LC were ligated together. The ligated DNA was transformed into DH5α competent cells and recovered for 1 hour. Some were collected and diluted for colony counting, and the rest were all plated on ampicillin-containing LB solid medium. Plated colonies were collected and stocked with 20% glycerol. As a result of titration of the transformed *E. coli* library, it was found that the library sizes were 2.98 × 10⁵ for HC and 5.64 × 10⁵ for LC.

**[Table 8] VH forward primers**

| Enzymatic site | VH forward region | Name | SEQ ID NO. |
|---|---|---|---|
| GGCCGCTA GGGCC | GAGGTTCDSCTGCAACAGTY | VH-for 1 | 80 |
| | CAGGTGCAAMTGMAGSAGTC | VH-for 2 | 81 |
| | GAVGTGMWGCTGGTGGAGTC | VH-for 3 | 82 |
| | CAGGTTAYTCTGAAAGAGTC | VH-for 4 | 83 |
| | GAKGTGCAGCTTCAGSAGTC | VH-for 5 | 84 |
| | CAGATCCAGTTSGYGCAGTC | VH-for 6 | 85 |
| | CAGRTCCAACTGCAGCAGYC | VH-for 7 | 86 |
| | GAGGTGMAGCTASTTGAGWC | VH-for 8 | 87 |
| | GAAGTGAAGMTTGAGGAGTC | VH-for 9 | 88 |
| | GATGTGAACCTGGAAGTGTC | VH-for 10 | 89 |
| | CAGATKCAGCTTMAGGAGTC | VH-for 11 | 90 |
| | CAGGCTTATCTGCAGCAGTC | VH-for 12 | 91 |
| | CAGGTTCACCTACAACAGTC | VH-for 13 | 92 |
| | CAGGTGCAGCTTGTAGAGAC | VH-for 14 | 93 |
| | GARGTGMAGCTGKTGGAGAC | VH-for 15 | 94 |

**[Table 9] VH reverse primer**

| VH reverse region | Name | SEQ ID NO. |
|---|---|---|
| CGAGGAGACGGTGACMGTGG | VH-rev 1 | 95 |
| CGCAGAGACAGTGACCAGAG | VH-rev 2 | 96 |
| CGAGGAGACTGTGAGASTGG | VH-rev 3 | 97 |

**[Table 10] CH forward primers**

| Portion overlapping with VH | CH1 forward portion | Name | SEQ ID NO. |
|---|---|---|---|
| CCA CKG TCA CCG TCT CCT CG | GCCTCCACCAAGGGCC CATCG | CH1_for 1 | 98 |
| CTC TGG TCA CTG TCT CTG CG | | CH1_for 2 | 99 |
| CCA STC TCA CAG TCT CCT CG | | CH1_for 3 | 100 |

**[Table 11] CH reverse primer**

| Sequence information | Name | SEQ ID NO. |
|---|---|---|
| CGAATTCAGAACCTCTTGGAACTAG CAAGT | pYDS_H_CH1_rev | 101 |

**[Table 12] Vk forward primers**

| Name | Homology region (pYDS-K) | Vκ forward region | TM | SEQ ID NO. |
|---|---|---|---|---|
| VL-for κ1 | GATAAAAGAG AGGCCGCTAG GGCC | G A C A W T G T T C T C A C C C A G T C | 52 | 102 |
| VL-for κ2 | | G A C A T C C A G A T G A C A C A G W C | 52 | 103 |
| VL-for κ3 | | G A T R T T G T G A T G A C C C A G W C | 50 | 104 |
| VL-for κ4 | | G A C A T T S T G M T G A C C C A G T C | 52 | 105 |
| VL-for κ5 | | G A T G T T G T G V T G A C C C A A A C | 50 | 106 |
| VL-for κ6 | | G A C A C A A C T G T G A C C C A G T C | 54 | 107 |
| VL-for κ7 | | G A Y A T T K T G C T C A C T C A G T C | 50 | 108 |
| VL-for κ8 | | G A T A T T G T G A T R A C C C A G G M | 49 | 109 |
| VL-for κ9 | | G A C A T T G T A A T G A C C C A | 48 | 110 |
| | | A T C | | |
| VL-for κ10 | | G A C A T T G T G A T G W C A C A G T C | 50 | 111 |
| VL-for κ11 | | G A T R T C C A G A T G A M C C A G T C | 51 | 112 |
| VL-for κ12 | | G A T G G A G A A A C A A C A C A G G C | 52 | 113 |

**[Table 13] Vk reverse primers**

| Name | Vk reverse region | CL region overhang | TM | SEQ ID NO. |
|---|---|---|---|---|
| Vk1/2_r ev | ACAGATGGTGCAGCCAC CGTACG | G C G T T T B A T T T C C A G C T T G G | 50 | 114 |
| VK3_rev | ACAGATGGTGCAGCCAC CGTACG | GCGTTTTATTTCCAA TTTTG | 44 | 115 |

**[Table 14] CL primers**

| Name | pYDS_K_CL | SEQ ID NO. |
|---|---|---|
| CL_for | CGTACGGTGGCTGCACCATCTGT | 116 |
| CL_rev | AATTACATGACTCGAGCTACTTGT CATCGTC | 117 |

### 4. Construction of Yeast Antibody Library

After a plasmid was prepared from the *E*. *coli* library, an antibody gene sequence was obtained by performing PCR using primers having the sequences shown in Table 15 below under the following conditions: 98°C for 3 min, and then 35 cycles, each consisting of 98°C for 30 sec, 55°C for 30 sec, and 72°C for 1 min, followed by 72°C for 3 min.

**[Table 15]**

| Primer name | Sequence information | SEQ ID NO. |
|---|---|---|
| PYDS_Seq_for | ccagcattgctgctaaagaagaaggg | 118 |
| PYDS_HC_Seq_Rev | cgaattcagaacctcttggaactagcaagt | 119 |
| PYDS_LC_Seq_Rev | atgactcgagctacttgtcatcgtcgt | 120 |

As competent cells, 200 µL of EBY100 yeast cells for HC and 200 µL of BJ5464 yeast cells for LC were placed in cuvettes, and then the antibody DNA and the sfil-treated pYDS vector at a ratio of 1:3 were added. Next, the cuvettes were placed in an electroporator and a pulse was applied to transform the yeast cells, followed by recovery for 1 hour. Some were collected and diluted for colony counting, and the rest were serially diluted on SDCAA + tryptophan (HC) and SDCAA + leucine (LC) agar plates. As a result of titration of the transformed yeast libraries, it was found that the library sizes were 2.98 × 10⁵ for HC and 5.64 × 10⁵ for LC.

The yeast HC library cells and the yeast LC library cells were mixed together in equal cell numbers (1 × 10⁹ cells), washed three times with YPD (20 g/L dextrose, 20 g/L peptone, 10 g/L yeast extract, 14.7 g/L sodium citrate, 4.29 g/L citric acid, pH 4.5) (SIGMA), and then resuspended with 100 µL of YPD. The cell suspension was dropped onto a YPD plate so as not to spread, and then dried and cultured at 30°C for 6 hours. After culture, the cells were collected in SD medium, centrifuged at 3,000 rpm for 3 minutes, and then washed. The cell pellet was dissolved to a total volume of 5 ml, and then serially diluted on SDCAA + tryptophan and SDCAA agar plates. The cells were subcultured in two SDCAA media in 500-mL Erlenmeyer flasks at an initial absorbance of 0.1. Culture was performed at 160 rpm and 30°C, and only the hybridized yeast was selected. The diversity of the hybridized yeast was shown to be 4 × 10⁷.

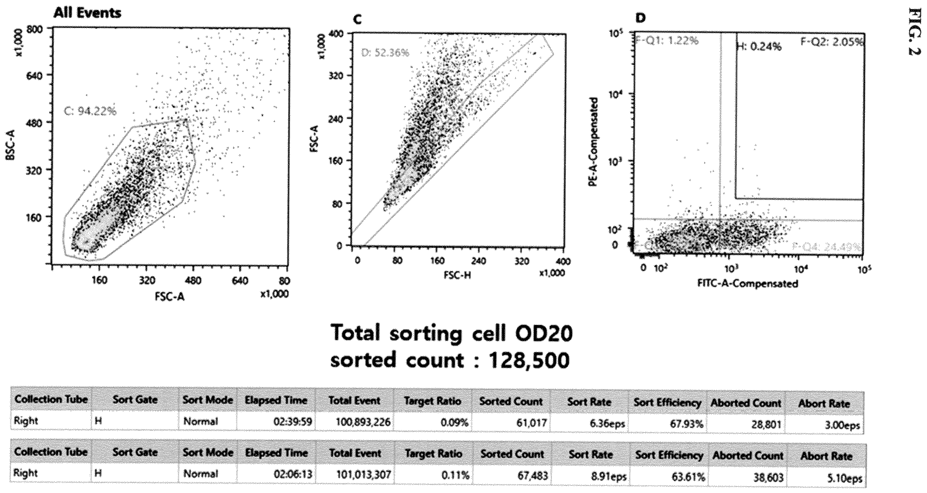

Yeast display antigen affinity selection was performed. Specifically, after inducing the yeast library, the absorbance at 600 nm was measured, and cells (OD 600nm = 20 (2 × 10⁸ cells)) to be used for FACS were taken, washed, and then resuspended in 1 mL of PBSF (0.1% BSA skim milk 1%) containing 1 µM of a mixture of 200:1 anti-frag (FG4R) and biotinylated MSLN-his. The cell suspension was incubated on a rotator (F1 mode, 25 speed) at room temperature for 1 hour and then washed. Second labeling was performed by adding anti-mouse IgG:FITC 200:1 (5 µL) and anti-human IgG:Fc-PE 200:1 (5 µL) in a 1-mL reaction volume (PBSF (BSA 0.1%)). After incubation on a rotator at 4°C for 15 minutes and washing, the cells were subjected to FACS sorting. Whether the antibody was expressed in yeast display was checked using the Flag tag of the heavy chain, and the affinity of the antibody to the antigen was examined using SA-PE, which binds to the biotin of the antigen. As a result, as shown in FIG. 2, 128,500 cells were selected by gating on yeast cells showing high reactivity with the antigen while showing Flag expression.

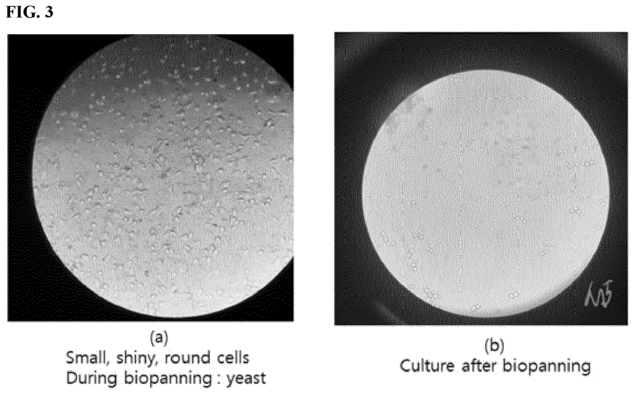

Yeast display antigen affinity biopanning was performed. Specifically, MSLN-positive Aspc1 cells and MSLN-negative Aspc1 cells were seeded in 6-well plates at 6×10⁵ cells per well and cultured. After 2 days, for biopanning, the cultured cells were fixed and washed. For positive selection, yeast was prepared at 1 × 10⁸ cells, 10⁷ cells, and 2 × 10⁷ cells per 3 wells. After washing the cells, the yeast pellet was resuspended in 1 mL of PBSF (0.1% BSA). PBS was completely removed from the cells in the 6-well plate, and the cells were washed once more with 1 mL of PBSF (0.1% BSA), and the supernatant was discarded. 0.5 mL of PBSF (0.1% BSA) was added to the cells, and 0.5 mL of the yeast cells were added dropwise to each well to achieve cell densities of 5×10⁷/mL, 2.5×10⁷/mL, and 1×10⁷/mL per well. The cells were incubated for 2 hours at 4°C while rotating on a rotator at 60 rpm. After incubation, the supernatant was discarded, and 1 mL of PBSF (0.1% BSA) was added to each well, and the plate was shaken 25 times and then rotated 5 times. The supernatant was discarded again, and 1 mL of PBSF (0.1% BSA) was added to each well. After shaking 25 times and rotating 10 times, the supernatant was discarded and PBSF (0.1% BSA) was added to each well. As a result of sterilizing the plate with quaternary ammonium and observing the same under a microscope, as shown in FIG. 3(a), it could be confirmed that the yeast bound well to the Aspc1 cells. Thereafter, PBSF in each well was discarded, 1 mL of SDCAA medium was added to each well, and the cells were harvested with a cell scraper and cultured overnight at 160 rpm at 30°C. As shown in FIG. 3(b), whether biopanning was achieved well was checked.

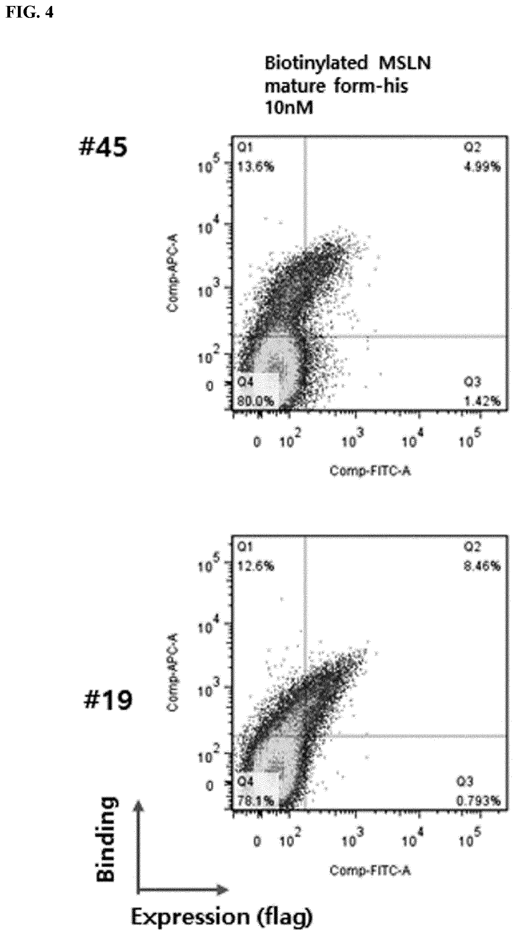

A single-cell antigen binding experiment was performed. A single colony was obtained from the plate and then inoculated into a 96-well plate containing a medium. After culturing overnight at 30°C and 160 rpm, the cells were induced at 20°C and 160 rpm for 48 hours. 4 × 10⁶ cells to be used for FACS were taken and transferred to a 96-well V-bottom plate. A total of 96 single clones were analyzed for their binding to the MSLN mature form-his antigen. After centrifugation at 3,000 rpm for 3 minutes, the supernatant was removed, 180 µL of PBSF (0.1% BSA) was added to each well, and the cells were resuspended 6 times or more and centrifuged at 3,000 rpm for 3 minutes, followed by removal of the supernatant. Thereafter, the cells were treated with 10 nM of MSLN mature form-his as an antigen and treated with anti-FLAG antibody (200:1). The 50 µL reaction volume was made using PBSF (0.1% BSA) and 1% skim milk. After mixing well, 50 µL was added to the centrifuged cell pellet which was then resuspended 6 times or more. The cell suspension was placed on a rocker, incubated at 50 rpm for 1 hour at room temperature, and washed. For second labeling, 50 µL anti-mouse IgG FITC (200:1) and 50 µL SA-APC (200:1) in a total of 10 mL of PBSF (0.1% BSA) were added to the cell suspension which was then incubated at 4°C for 15 minutes, washed, and subjected to FACS analysis. As a result, as shown in FIG. 4, it could be confirmed that clone #19 and clone #45 bound to the mature form of MSLN.

### 5. Purification of scFv from Selected Single Cells

After FACS analysis, single cells of clone #19 and clone #45 were added into and lysed in 0.2% SDS solution at 95°C for 20 minutes. After spinning down, the supernatant was used as a template. Using 1 µL of this template, 20 pmole forward primer shown in Table 16 below, 20 pmole reverse primer shown in Table 16 below, 4 µL of 10 mM dNTP, 5 µL of 10x pfu buffer, and 1 µL of Pfu DNA pol, a total of 40 µL of a PCR mixture was prepared. Using the PCR mixture, PCR was performed under the following conditions: 94°C for 3 min, and then 35 cycles, each consisting of 94°C for 30 sec, 55°C for 30 sec, and 72°C for 1 min, followed by 72°C for 3 min. The DNA band was gel-extracted and sequence information of the DNA was obtained by the Sanger sequencing technique. The DNA sequence was translated to the amino acid sequence, and the antibody sequence information was confirmed using the IMGT/DomainGapAlign web program (https://www.imgt.org/3Dstructure-DB/cgi/DomainGapAlign.cgi). The results are shown in Table 17 below. (For reference, the sequence information below is based on IMGT numbering.)

**[Table 16]**

| Primer | Name | Sequence information | SEQ ID NO. |
|---|---|---|---|
| Forward primer for HC and LC | pYDSH_colonyPCR_ for | CACTATCCAACAGCACAAATA ACGGGTTATCT | 121 |
| Reverse primer for HC | BCRHC_CH1_Rev | GGAGGAGGGTGCCAGGGGGA | 122 |
| Reverse primer for | pYDSK_CL_Rev | TGTACTTTGGCCTCTCTGGGAT | 123 |
| LC | | AGAAGTTATT | |

**[Table 17]**

| Clone | Classification | | Sequence information | SEQ ID NO. |
|---|---|---|---|---|
| #19 | VH | Amino acid sequence | QVQLQQSGPELVKPGASVKISCKASGYSFTDYN MNWVKQSNGKSLEWIGVINPNYGTTSYNQKFK GKATITADTSSNTAYLQLSSLTSEDSAVYYCAKG DYDGAGFAYWGQGTLVTVSA | 38 |
| | VL | | DIVITQSPAIMSASPGEKVTMTCSASSSVSYMHW YQQKSGTSPKRWIYDTSKLASGVPARFSGSGSG TSYSLTISSMEAEDAATYYCQQWSSNPLTFGAG TKLEIKR | 39 |
| | VH | Nucleic acid sequence | CAGGTCCAACTGCAGCAGTCTGGACCTGAGCT GGTGAAGCCTGGCGCTTCAGTGAAGATATCCT GCAAGGCTTCTGGTTACTCATTCACTGACTAC AACATGAACTGGGTGAAGCAGAGCAATGGAA AGAGCCTTGAGTGGATTGGAGTAATTAATCCT AACTATGGTACTACTAGCTACAATCAGAAGTT CAAGGGCAAGGCCACTATAACAGCAGACACA TCCTCCAACACAGCCTACCTGCAGCTCAGCAG CCTGACATCTGAGGACTCTGCAGTCTATTACT GTGCAAAGGGTGATTACGACGGGGCCGGGTTT GCTTACTGGGGCCAAGGGACTCTGGTCACTGT CTCTGCG | 40 |
| | VL | | GATATTGTGATAACCCAGTCTCCAGCAATCAT | 41 |
| | | | GTCTGCATCTCCAGGGGAGAAGGTCACCATGA CCTGCAGTGCCAGCTCAAGTGTAAGTTACATG CACTGGTACCAGCAGAAGTCAGGCACCTCCCC CAAAAGATGGATTTATGACACATCCAAACTGG CTTCTGGAGTCCCTGCTCGCTTCAGTGGCAGT GGGTCTGGGACCTCTTACTCTCTCACAATCAG CAGCATGGAGGCTGAAGATGCTGCCACTTATT ACTGCCAGCAGTGGAGTAGTAACCCACTCACG TTCGGTGCTGGGACCAAGCTGGAAATCAAACG C | |
| #45 | VH | Amino acid sequence | QVQLQQPGAELVKPGASVKLSCKASGYTFTSY WMHWVKQRPGQGLEWIGMIHPNSGGTNYNEK FKSKATLTVDKSSSTAYMQLSSLTSEDSAVYYC ARYYYGNYVWYFDVWGTGTTVTVSA | 42 |
| | VL | | DIVLTQSPAIMSTSPGEKVTMTCTASSSVSYMH WYQQKSGTSPKRWIYDTSKLASGVPARFSGSGS GTSYSLTISRMEAEDAATYYCQQWSSYPLTFGA GTKLEMKR | 43 |
| | VH | Nucleic acid sequence | CAGGTCCAACTGCAGCAGCCTGGGGCTGAACT GGTGAAGCCTGGGGCTTCAGTGAAGCTGTCCT GCAAGGCTTCTGGCTACACCTTCACCAGCTAC TGGATGCACTGGGTGAAGCAGAGGCCTGGAC AAGGCCTTGAGTGGATTGGAATGATTCATCCT AATAGTGGTGGTACTAACTACAATGAGAAGTT CAAGAGCAAGGCCACACTGACTGTAGACAAA | 44 |
| | | | TCCTCCAGCACAGCCTACATGCAACTCAGCAG CCTGACATCTGAGGACTCTGCGGTCTATTACT GTGCAAGATACTACTATGGTAACTACGTGTGG TACTTCGATGTCTGGGGCACAGGGACCACGGT CACCGTCTCTGCG | |
| | VL | | GACATTGTTCTCACCCAGTCTCCAGCAATCAT GTCTACATCTCCGGGGGAGAAGGTCACCATGA CCTGCACTGCCAGCTCAAGTGTAAGTTACATG CACTGGTACCAGCAGAAGTCAGGCACCTCCCC CAAAAGATGGATTTATGACACATCCAAACTGG CTTCTGGAGTCCCTGCTCGCTTCAGTGGCAGT GGGTCTGGGACCTCTTACTCTCTCACAATCAG CCGAATGGAGGCTGAAGATGCTGCCACTTATT ACTGCCAGCAGTGGAGTAGTTACCCACTCACG TTCGGTGCTGGGACCAAGCTGGAAATGAAACG C | 45 |

After sequencing as described above, PCR was performed on each clone so that the HC and LC and the pmopac12 vector could overlap each other. The HC, LC and pmpac12 vector PCR products were added to the Gibson assembly mixture, and then allowed to react at 50°C for 200 minutes. The reaction products were cloned into DH5α competent cells, and then scFv expression vectors of antibody clone #19 and antibody clone #45 were collected. Co-transformation of BL21 cells with a pET vector containing the BirA gene was performed. After culturing in a medium containing kanamycin and chloramphenicol, the cells were grown starting from an initial cell concentration of 5 × 10⁵ cells/mL to 6-8 × 10⁶ cells/ml, and then IPTG was added thereto at a concentration of 0.5 mM. The cells were induced at 25°C and 160 rpm for 16 hours.

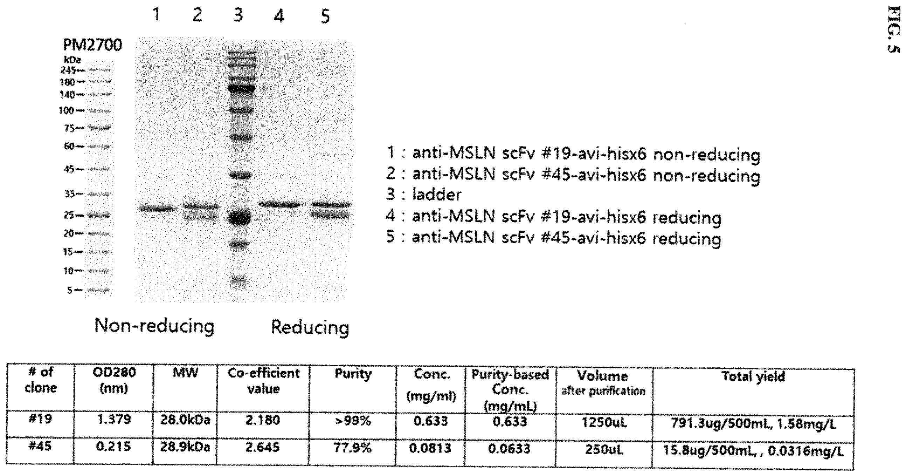

After induction, the cells were centrifuged at 5,000 xg at 4°C for 10 minutes, and then the supernatant was discarded. Per 50 mL of induction volume, 320 µL of a lysozyme solution (containing 20 mg/mL lysozyme, 100 mM Tris, 0.75 M sucrose, pH 7.5) was added to the pellet which was then resuspended. Thereafter, the lysozyme solution was additionally added to the suspension which was then incubated with shaking at 4°C for 20 minutes while rotating at 100 rpm. Thereafter, 1 mM EDTA was slowly added dropwise to the suspension in an amount of 6.25 mL per 50 mL of induction volume, and the resulting suspension was incubated with shaking at 4°C for 20 minutes while rotating at 100 rpm. After centrifugation at 12,000 xg for 30 minutes, the supernatant was filtered through a 0.22-µm PES syringe filter. The supernatant was loaded onto Ni-NTA resin, and 15 mL of washing buffer (25 mM imidazole, 300 mM NaCl, 50 mM NaH₂PO₄, pH8.0) was flowed, and then 15 mL of washing buffer (25 mM imidazole, 300 mM NaCl, 50 mM NaH₂PO₄, pH 8.0, 0.05% Triton x-114) was flowed. After flowing 15 mL of washing buffer (25 mM imidazole, 300 mM NaCl, 50 mM NaH₂PO₄, pH 8.0), 3 mL of elution buffer (250 mM imidazole, 300 mM NaCl, 50 mM NaH₂PO₄, pH 8.0) was flowed so that total of 15 mL was flowed. The eluate was recovered. The eluate was transferred to an Amicon Ultra 15mL centrifugal filter (10 kDa) and then centrifuged at 3,500 rpm for 30 minutes. Once concentrated, the buffer was replaced, the OD value at 280 nm was measured using a NanoDrop, and the actual concentration of the protein was calculated. Next, 1 µg of the protein was subjected to SDS-PAGE analysis to measure the purity, and then the concentration of scFv protein was calculated. As a result, as shown in FIG. 5, it could be found that scFv with a molecular weight of approximately 28 to 29 kDa was purified.

### [Example 21 Evaluation of Binding Capacity of Mesothelin-Specific Antibody

### 1. ELISA-Based Evaluation of Antigen Binding Capacity

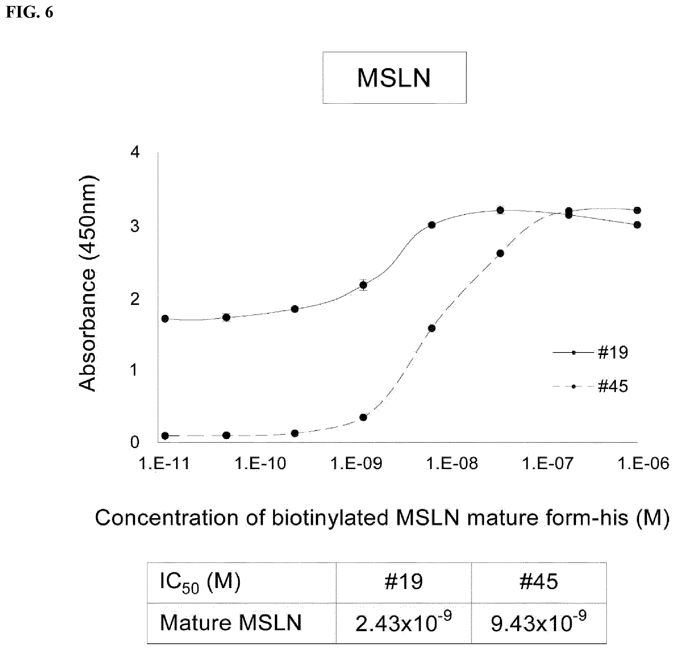

100 ng of the scFv protein purified in Example 1 was added to each well of a plate and refrigerated for 15 hours, thereby coating the plate with the protein. After incubation, each well was blocked with 200 µL of PBS (3% BSA) for 1 hour at room temperature. Biotinylated MSLN-his protein was serially diluted 1/5 from 10⁻⁶ M and then bound to the anti-MSLN scFv, coated on the ELISA plate, for 1 hour at room temperature. Each well was washed three times with PBST (0.05% tween 20) wash buffer, and then treated with HRP-conjugated streptavidin (Abcam) (diluted 40,000:1) for detection of biotinylated antibody and incubated at room temperature for 1 hour. Each well was washed three times with PBST (0.05% tween20) wash buffer, and then treated with TMB and incubated at room temperature for 20 minutes. After treatment with 2M H₂SO₄, the absorbance at 450 nm was measured. As a result, as shown in FIG. 6, it could be confirmed that the anti-MSLN scFv antibodies prepared according to the present invention had excellent binding capacity to the MSLN antigen.

### 2. Evaluation of Antigen Binding Capacity Using MSLN-Expressing Cells

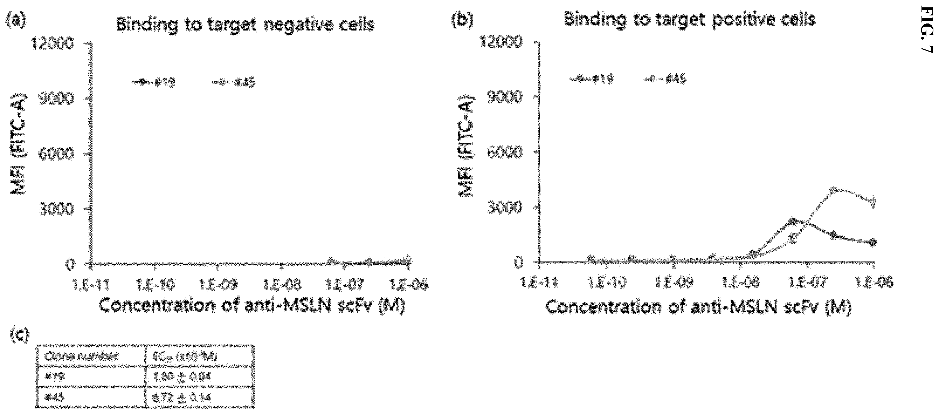

For each of the purified scFv antibodies, their binding capacity to the MSLN antigen was analyzed using cells expressing MSLN. Specifically, MSLN-positive K562 cells and MSLN-negative K562 cells were prepared and inoculated into 96-well plates at a density of 5 × 10⁴ cells per well. After washing with PBSF (1% FBS) wash buffer, each well was incubated with the anti-MSLN scFv (serially diluted 1/4 from 10⁻⁶ M) according to the present invention at 4°C for 1 hour. Each well was washed with PBSF (1% FBS) wash buffer. Thereafter, each well was treated with anti-HISx6-FITC (250:1) and incubated at 4°C for 1 hour. After washing with PBSF (1% FBS), the cells were subjected to FACS analysis. As a result, as shown in FIG. 7, it could be confirmed that the anti-MSLN scFv antibodies prepared according to the present invention bound selectively to the MSLN-positive K562 cells without binding to the MSLN-negative K562 cells.

### [Example 3] Generation of NK Cells Expressing Mesothelin-Specific Chimeric Antigen Receptor (MSLN-CAR)

### 1. Construction of Mesothelin-Specific Chimeric Antigen Receptor Construct Expression Vector

10 µg of a pNKLV3 vector to be used for virus production was treated with XbaI (NEB, cat##R0145S) and BamHI-HF (NEB, cat##R3136S) restriction enzymes to make a linear form. As the chimeric antigen receptor construct shown in FIG.8, a CAR DNA composed of a polynucleotide (SEQ ID NO: 58) encoding a CD8a signal peptide, a polynucleotide (#19 clone: SEQ ID NO: 52, #45 clone: SEQ ID NO: 54) encoding a mesothelin-binding scFv, a polynucleotide (SEQ ID NO: 60) encoding a CD8 hinge region, a polynucleotide (SEQ ID NO: 62) encoding a CD8 transmembrane domain, a polynucleotide (SEQ ID NO: 64) encoding 4-1BB (co-stimulatory domain), and a polynucleotide (SEQ ID NO: 66) encoding CD3ζ (intracellular signaling domain) was designed and synthesized *in vitro* so that approximately 12 bp of DNA at the 5' and 3' ends were identical to the linear pNKLV3 vector. 100 ng of the linear pNKLV3 vector and 50 ng of the synthesized CAR construct DNA were mixed with 5x Ez-fusion HT cloning PreMIX (Enzynomics, cat# EZ015TL) and ligated together at 50°C.

### 2. Production of Mesothelin-Specific Chimeric Antigen Receptor Construct Virus

Virus production was performed using the LV-MAX transfection kit (Thermofisher, cat# A35348). 25.5 mL of cultured HEK293F cells were prepared at 4.7 × 10⁶ cells/mL, and then 1.5 mL of LV-MAX supplement was added and the cells were cultured in a shaking incubator at 37°C and 120 rpm under 8% CO₂. Four different vectors, that is, the vector constructed in Example 3-1 above, pMDLg/pRRE (Addgene, cat# 12251) pMD2.G (Addgene, cat# 12259), and pRSV-Rev (Addgene, cat# 12253), were placed in a 50-mL tube in amounts of 33.33 µg, 16.67 µg, 16.67 µg, and 8.33 µg, respectively, and the volume was adjusted to 1.5 mL with Opti-MEM medium. 1,320 µL of Opti-MEM (Gibco, Cat# 11058021) medium and 180 µL of reagent were added to a fresh tube and then left to stand for 1 minute. The solutions in the tube containing the vectors and the tube containing the reagent were mixed together and then left to stand for 10 minutes. HEK293F cells in culture were taken out, added to the mixed reagent, and cultured in a shaking incubator for 5 hours at 37°C and 120 rpm under 8% CO₂. After culturing, 1.2 mL of enhancer was added to the cells which were then cultured for 2 days, and then the cells were removed, thereby producing a virus.

### 3. Generation of NK Cells Expressing Mesothelin-Specific CAR

Natural killer cells were used as immune effector cells to be genetically engineered to express the chimeric antigen receptor. As such natural killer cells, NK-92 cells were purchased from ATCC. NK-92 cells and the lentivirus-transduced NK-92 cells described below were all cultured in media containing 100 µg/mL of streptomycin, 100 units/mL of penicillin, 20% fetal bovine serum (FBS), 10% MEM vitamin solution, 1X 2-mercaptoethanol, and 200 ill of rhIL-2. For activation, NK-92 cells were cultured in medium containing 500 IU of rhIL-2 and 10 ng/mL of rhIL-15 2 days before transduction. 293T cells were cultured in medium containing 100 µg/mL of streptomycin, 100 units/mL of penicillin, and 10% fetal bovine serum (FBS), and all cells were cultured in a 5% CO₂ (95% air) environment at 37°C. For expression of the mesothelin-specific CAR, 5 × 10⁵ NK-92 cells and the produced antigen receptor construct virus with an MOI of 10 were added to a 24-well plate, and to induce transduction, centrifugation was performed at 1000xg for 60 minutes, thereby generating NK cells expressing the mesothelin-specific CAR.

### 4. Selection of Cells Expressing Mesothelin-Specific CAR

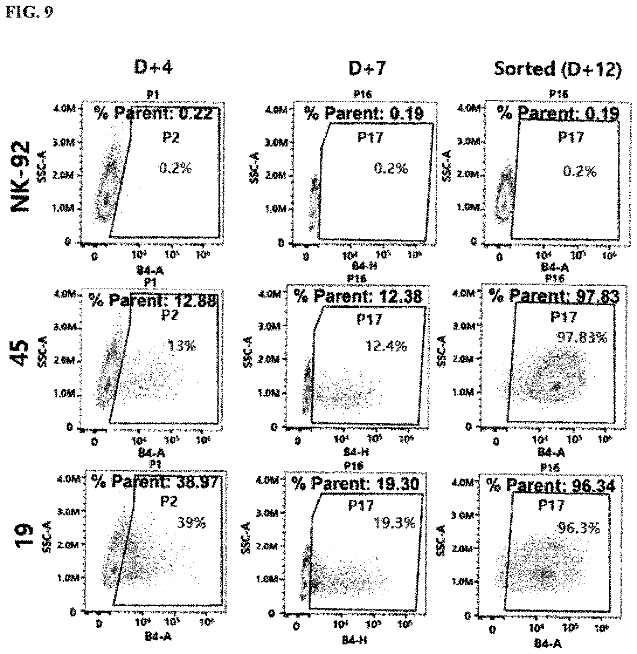

In order to select only cells expressing mesothelin-specific CAR, labeling with the biotinylated mesothelin of SEQ ID NO: 1 was performed and then magnetic anti-biotin microbeads were added. Therefore, unlabeled cells passed through the MACS column in a magnetic field, while magnetically labeled cells were retained in the column, and thus only cells expressing mesothelin-specific CAR could be selected. After culturing the selected cells for a total of 12 days, FACS analysis was performed. As a result, as shown in FIG. 9, it could be confirmed that the mesothelin-specific CAR comprising scFv #19 or scFv #45 was expressed in 97% or more of the selected cells.

### 5. Phenotypic Analysis of Cells Expressing Mesothelin-Specific CAR

In order to analyze the phenotype of the cells selected in Example 3-4 above, the cells were rinsed twice with FACS buffer, and the rinsed cells were subjected to FACS analysis using PE-conjugated anti-CD56 antibody, PE-conjugated anti-CD16 antibody, PE-conjugated anti-NKG2D antibody, PE-conjugated anti-NKp30 antibody, PE-conjugated anti-LDLR antibody, and fluorescent dye-conjugated MSLN antigen.

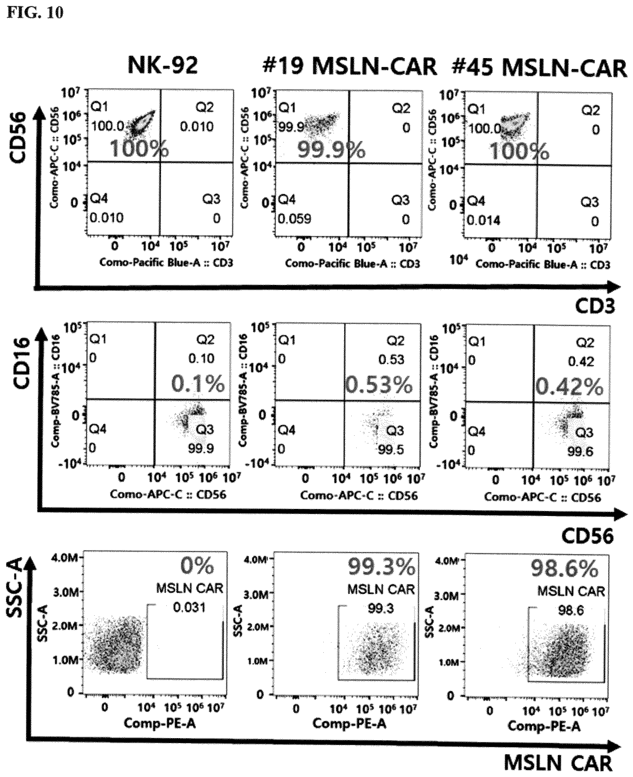

As a result, as shown in FIG. 10, it could be confirmed that the cells selected in Example 3-4 above were CD56⁺CDI6⁻ NK-92 cells and the mesothelin-specific CAR was expressed in 99% or more of the cells. In addition, it could be confirmed that NKG2D, an activating receptor for NK cells, was expressed at a higher level in the CAR-expressing NK cells than in the negative control group (NK-92 cells).

### [Example 41 Evaluation of Cancer Cell Killing Ability of NK Cells Expressing Mesothelin-Specific Chimeric Antigen Receptor (CAR)

The cells selected in Example 3-4 above were co-cultured with Capan-2 pancreatic cancer cells, known to highly express mesothelin, at a cell number ratio of 2:1. After 4 hours of co-culture, dead or damaged cells were stained with PI (propidium iodide). Next, the killing ability of the CAR-expressing NK cells against the cancer cells was analyzed by FACS.

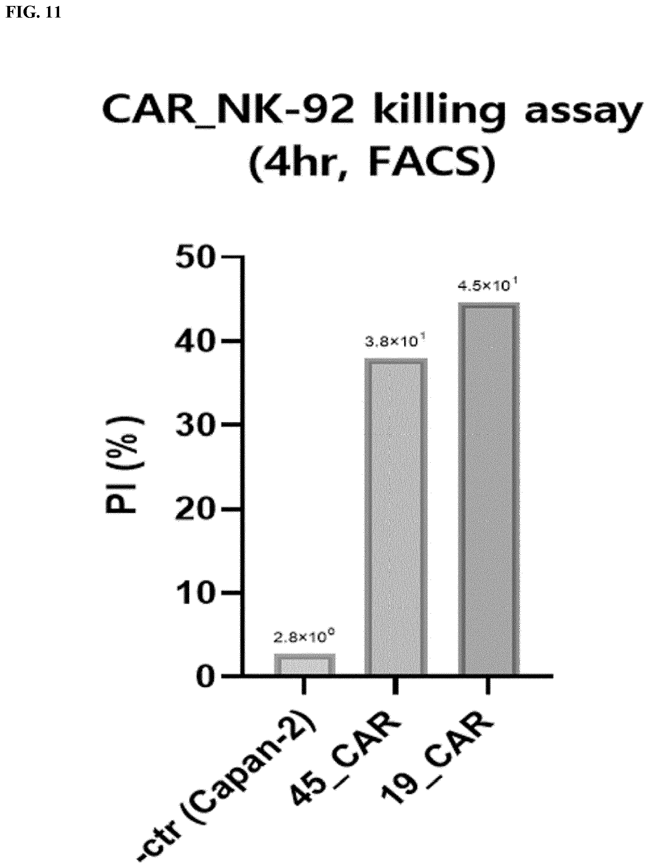

As shown in FIG. 11, it could be confirmed that the mesothelin-specific CAR-expressing NK cells according to the present invention had excellent killing ability against the mesothelin-expressing pancreatic cancer cells.

### [Example 51 Induction of Mesothelin-Specific Humanized Antibody

A humanized antibody was prepared by changing the mouse scFv #19 selected in Example 1 to a structure corresponding to that of a human. Specifically, deep learning on natural antibody repertoires (Sapiens) or CDR grafting was performed using the BioPhi site (https://biophi.dichlab.org/humanization/humanize/), and humanized antibody sequence substitution was performed focusing on the human IGHV1-46 and IGKV3-11 framework regions. According to the OASis percentile standard (MAbs. 2022; 14(1): 2020203.), the sequence was substituted to be 37-40% or more, which is the standard value for humanized antibody sequences. The "OASis prevalence threshold", a setting variable for evaluating humanized antibody sequences, was set to "strict (≥90% subjects)" and mouse scFv #19 was obtained as a humanized antibody sequence (OASis percentile value: 58%). Here, the humanized antibody was named humanized antibody #19-1. The amino acid and nucleotide sequences of the heavy chain variable region and light chain variable region of the antibody are shown in Table 18 below. (For reference, the following sequence information is based on IMGT/DomainGapAlign web program (https://www.imgt.org/3Dstructure-DB/cgi/DomainGapAlign.cgi) IMGT numbering.)

**[Table 18]**

| #19-1 | Sequence information | SEQ ID NO. |
|---|---|---|
| Heavy chain | GYTFTDYN | 29 |
| variable region CDR1 | | |
| Heavy chain variable region CDR2 | INPNYGTT | 5 |
| Heavy chain variable region CDR3 | ARGDYDGAGFAY | 32 |
| Light chain variable region CDR1 | SSVSY | 10 |
| Light chain variable region CDR2 | DTS | 12 |
| Light chain variable region CDR3 | QQWSSNPLT | 14 |
| Amino acid sequence of heavy chain variable region | QVQLVQSGAEVKKPGASVKVSCKASGYTFTDYNM NWVRQAPGQGLEWMGVINPNYGTTSYNQKFKGR VTMTRDTSTSTVYMELSSLRSEDTAVYYCARGDYD GAGFAYWGQGTLVTVSS | 46 |
| Amino acid sequence of light chain variable region | EIVLTQSPATLSLSPGERATLSCSASSSVSYMHWYQ QKPGQAPRLLIYDTSKLASGIPARFSGSGSGTDFTLT ISSLEPEDFAVYYCQQWSSNPLTFGQGTKLEIKR | 47 |
| Nucleotide sequence of heavy chain variable region | CAGGTCCAACTGGTTCAGTCTGGAGCTGAGGTAA AGAAGCCTGGCGCTTCAGTGAAGGTTTCCTGCAA GGCTTCTGGTTACACCTTCACTGACTACAACATG AACTGGGTGCGACAGGCTCCCGGACAAGGACTT GAGTGGATGGGAGTAATTAATCCTAACTATGGTA CTACTAGCTACAATCAGAAGTTCAAGGGCAGGGT GACTATGACACGCGACACATCCACCTCTACAGTG TACATGGAGCTCAGCAGCCTGCGCTCTGAGGACA CGGCAGTCTATTACTGTGCAAGAGGTGATTACGA CGGGGCCGGGTTTGCTTACTGGGGCCAAGGGACT CTGGTCACTGTCTCTAGC | 48 |
| Nucleotide sequence of light chain variable region | GAAATTGTcttAACCCAGTCTCCAGCAACCCTTTCT TTGTCTCCAGGGGAGCGAGCAACCCTGAGTTGCA GTGCCAGCTCAAGTGTAAGTTACATGCACTGGTA CCAGCAGAAGCCTGGCCAAGCGCCCCGCCTTCTC ATTTATGACACATCCAAACTGGCTTCTGGAATAC CTGCTCGCTTCAGTGGCAGTGGGTCTGGGACCGA CTTTACTCTCACAATCAGCAGCTTGGAGCCAGAA GATTTTGCCGTATATTACTGCCAGCAGTGGAGTA GTAACCCACTCACGTTCGGTCAGGGGACCAAGCT GGAAATCAAACGC | 49 |

### [Example 61 Expression and Purification of Mesothelin-Specific Humanized ScFv

The humanized antibody #19-1 sequence predicted in Example 5 above was expressed in a mammal and then purified. First, to optimize expression efficiency, the gene sequence of scFv antibody #19-1 was optimized using the codon optimization tool (https://sg.idtdna.com/CodonOpt). For purification, a gene was synthesized in which a human IgG-Fc tag was linked to the 3' end of the scFv sequence. The synthesized gene was inserted into the pCEP4 mammalian expression vector and expressed in the form of #19-1 scFv-Fc using the ExpiCHO Expression System. Subsequently, purification was performed using protein A affinity chromatography and size exclusion chromatography techniques.

### [Example 7] Evaluation of Binding Affinity of Humanized Antibody to Mesothelin

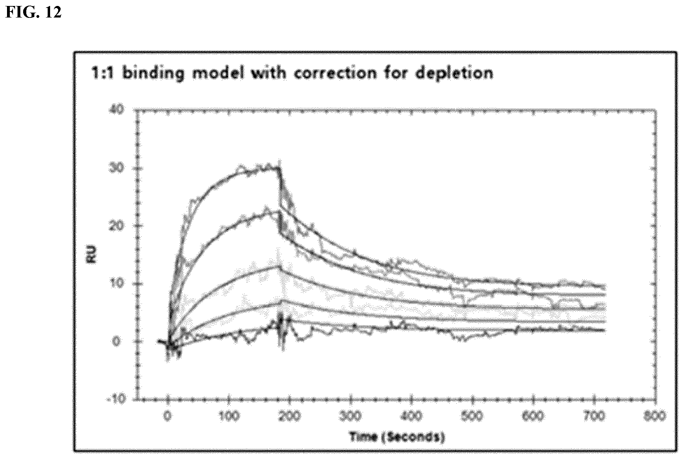

The binding affinity of the humanized antibody #19-1 purified in Example 6 to mesothelin was evaluated by surface plasmon resonance (SPR) method. First, recombinant human mature MSLN (SEQ ID NO: 1) was immobilized by an amide bond on the surface of a carboxymethyldextran (CM5) chip at densities of about 560 RU and 520 RU. Next, two-fold serial dilutions of humanized antibody #19-1 were injected at a constant flow rate. The association and dissociation rates of the protein complex (complex of humanized antibody #19-1 and mature MSLN) were monitored for 180 and 540 seconds, respectively. Referencing was performed on a blank flow cell. Finally, the binding affinity of humanized antibody #19-1 to mesothelin was determined using the 1:1 depletion corrected binding model. The analysis results are shown in FIG. 12 and Table 19 below.

**[Table 19]**

| | MSLN |
|---|---|
| Humanized antibody #19-1 | 80.8 nM |

As shown in Table 19 above, the dissociation constant (KD) of humanized antibody #19-1 of the present invention was 80.8 nM, suggesting that the binding affinity to mesothelin was high.

### [Example 8] Generation of Cells Expressing Mesothelin-Specific Chimeric Antigen Receptor (MSLN-CAR)

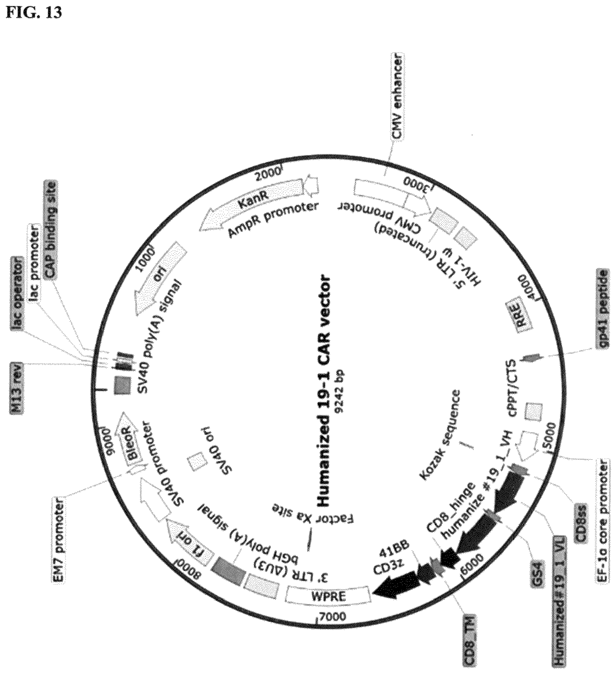

As shown in FIG. 13, as a chimeric antigen receptor expression cassette, a polynucleotide encoding a CD8 signal peptide, a mesothelin binding domain (humanized scFv), a CD8 hinge, a CD8 transmembrane domain, 4-1BB, and a CD3 zeta signaling domain was inserted into a lentiviral transfer vector. At this time, the recombinant gene was placed under the control of the EF1a single promoter. The nucleotide sequence of the EF1a promoter used in the experiment and the nucleotide sequences of each other gene are shown in Table 20 below, and the amino acid sequences encoded by these nucleotide sequences are shown in Table 21 below.

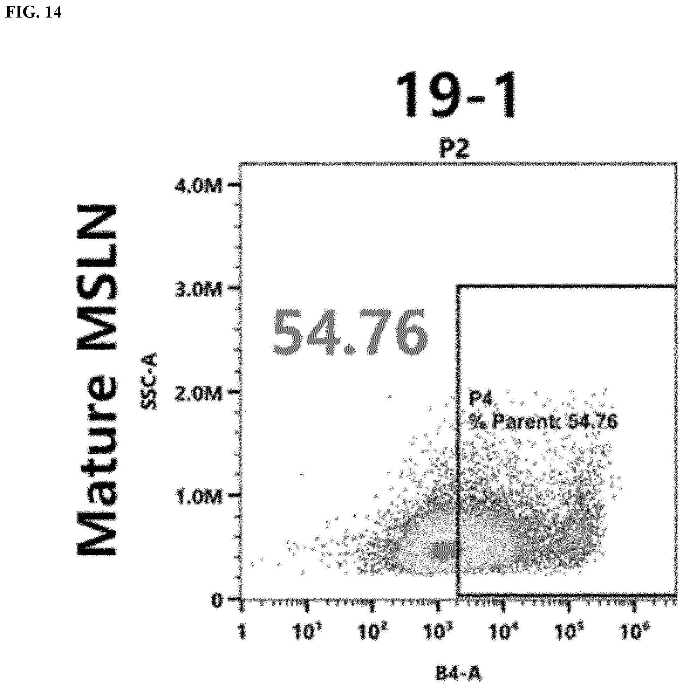

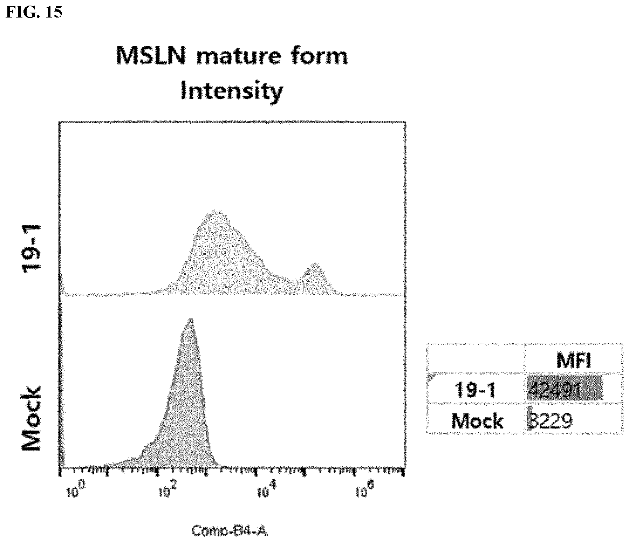

293T cells were used as cells to be genetically engineered to express the chimeric antigen receptor. These 293T cells were purchased from ATCC. 293T cell line and the lentivirus-transduced 293T cells described below were all cultured in DMEM (Dulbecco's Modified Eagle Medium) containing 100 µg/mL of streptomycin, 100 units/mL of penicillin, and 10% fetal bovine serum (FBS), and culture of the cells were performed in a 5% CO₂ (95% air) environment at 37°C. For expression of the mesothelin-specific CAR, 5 × 10⁵ 293T cells and the produced antigen receptor construct virus with an MOI of 10 were added to a 24-well plate, and then for induction of transduction, centrifugation was performed at 1,000xg for 60 minutes, thereby generating 293T cells expressing the mesothelin-specific CAR. The generated cells were cultured for 2 days. To examine the binding efficiency and intensity of the mesothelin-specific CAR-expressing 293T cells to mesothelin, the cells were first rinsed twice with FACS buffer, and the biotinylated mature form of the mesothelin antigen was bound to the rinsed cells. Then, biotin was fluorescently labeled using PE-conjugated streptavidin protein and FACS analysis was performed. As a result, as shown in FIGS. 14 and 15, it could be confirmed that approximately 55% of the 293T cells expressing the mesothelin-specific CAR bound to the mesothelin antigen, and the binding intensity thereof was also high.

**[Table 20]**

| Gene name | Nucleotide sequence | SEQ ID NO. |
|---|---|---|
| EF1a promoter | gggcagagcgcacatcgcccacagtccccgagaagttggggggaggggtcggcaattgatccg gtgcctagagaaggtggcgcggggtaaactgggaaagtgatgtcgtgtactggctccgcctttttc ccgagggtgggggagaaccgtatataagtgcagtagtcgccgtgaacgttctttttcgcaacgggtt tgccgccagaacacag | 67 |
| CD8 signal peptide | ATGGCCTTACCAGTGACCGCCTTGCTCCTGCCGCTGGCCTT GCTGCTCCACGCCGCCAGGCCG | 58 |
| scFv | GAAATTGTCTTAACCCAGTCTCCAGCAACCCTTTCTTTGTCT CCAGGGGAGCGAGCAACCCTGAGTTGCAGTGCCAGCTCAA GTGTAAGTTACATGCACTGGTACCAGCAGAAGCCTGGCCAA GCGCCCCGCCTTCTCATTTATGACACATCCAAACTGGCTTCT GGAATACCTGCTCGCTTCAGTGGCAGTGGGTCTGGGACCGA CTTTACTCTCACAATCAGCAGCTTGGAGCCAGAAGATTTTG CCGTATATTACTGCCAGCAGTGGAGTAGTAACCCACTCACG TTCGGTCAGGGGACCAAGCTGGAAATCAAACGCGGAGGTG GGGGCAGTGGAGGCGGTGGTAGTGGAGGCGGAGGAAGTCA GGTCCAACTGGTTCAGTCTGGAGCTGAGGTAAAGAAGCCTG GCGCTTCAGTGAAGGTTTCCTGCAAGGCTTCTGGTTACACC TTCACTGACTACAACATGAACTGGGTGCGACAGGCTCCCGG ACAAGGACTTGAGTGGATGGGAGTAATTAATCCTAACTATG GTACTACTAGCTACAATCAGAAGTTCAAGGGCAGGGTGACT | 56 |
| | ATGACACGCGACACATCCACCTCTACAGTGTACATGGAGCT CAGCAGCCTGCGCTCTGAGGACACGGCAGTCTATTACTGTG CAAGAGGTGATTACGACGGGGCCGGGTTTGCTTACTGGGGC CAAGGGACTCTGGTCACTGTCTCTAGC | |
| CD8 hinge | ACCACGACGCCAGCGCCGCGACCACCAACACCGGCGCCCA CCATCGCGTCGCAGCCCCTGTCCCTGCGCCCAGAGGCGTGC CGGCCAGCGGCGGGGGGCGCAGTGCACACGAGGGGGCTGG ACTTCGCCTGTGAT | 60 |
| CD8 transmembr ane | ATCTACATCTGGGCGCCCTTGGCCGGGACTTGTGGGGTCCT TCTCCTGTCACTGGTTATCACCCTTTACTGC | 62 |
| 4-1BB | AAACGGGGCAGAAAGAAACTCCTGTATATATTCAAACAAC CATTTATGAGACCAGTACAAACTACTCAAGAGGAAGATGG CTGTAGCTGCCGATTTCCAGAAGAAGAAGAAGGAGGATGT GAACTG | 64 |
| CD3 zeta | AGAGTGAAGTTCAGCAGGAGCGCAGACGCCCCCGCGTACA AGCAGGGCCAGAACCAGCTCTATAACGAGCTCAATCTAGG ACGAAGAGAGGAGTACGATGTTTTGGACAAGAGACGTGGC CGGGACCCTGAGATGGGGGGAAAGCCGAGAAGGAAGAAC CCTCAGGAAGGCCTGTACAATGAACTGCAGAAAGATAAGA TGGCGGAGGCCTACAGTGAGATTGGGATGAAAGGCGAGCG CCGGAGGGGCAAGGGGCACGATGGCCTTTACCAGGGTCTC AGTACAGCCACCAAGGACACCTACGACGCCCTTCACATGCA GGCCCTGCCCCCTCGC | 66 |

**[Table 21]**

| Peptide name | Amino acid sequence | SEQ ID NO. |
|---|---|---|
| CD8 signal peptide | MALPVTALLLPLALLLHAARP | 57 |
| scFv | EIVLTQSPATLSLSPGERATLSCSASSSVSYMHWYQQKP GQAPRLLIYDTSKLASGIPARFSGSGSGTDFTLTISSLEP EDFAVYYCQQWSSNPLTFGQGTKLEIKRGGGGSGGGG SGGGGSQVQLVQSGAEVKKPGASVKVSCKASGYTFTD YNMNWVRQAPGQGLEWMGVINPNYGTTSYNQKFKG RVTMTRDTSTSTVYMELSSLRSEDTAVYYCARGDYDG AGFAYWGQGTLVTVSS | 55 |
| CD8 hinge | TTTPAPRPPTPAPTIASQPLSLRPEACRPAAGGAVHTRG LDFACD | 59 |
| CD8 transmembran e | IYIWAPLAGTCGVLLLSLVITLYC | 61 |
| 4-1BB | KRGRKKLLYIFKQPFMRPVQTTQEEDGCSCRFPEEEEG GCEL | 63 |
| CD3 zeta | RVKFSRSADAPAYKQGQNQLYNELNLGRREEYDVLDK RRGRDPEMGGKPRRKNPQEGLYNELQKDKMAEAYSEI GMKGERRRGKGHDGLYQGLSTATKDTYDALHMQALP PR | 65 |

### [Example 9] Generation of NK Cells Expressing Mesothelin-Specific Chimeric Antigen Receptor (MSLN-CAR)

A mesothelin-specific chimeric antigen receptor construct virus was produced in the same manner as in Example 8 above, and then natural killer cells were used as immune effector cells to be genetically engineered to express the chimeric antigen receptor. As such natural killer cells, PBMC (Human PB MNC) cells were purchased from StemCell, cultured for 10 days, and used after differentiation into PBNK cells. PBMC and PBNK cells, as well as lentivirus-transduced PBNK cells described below, were all cultured in NK MACS medium containing 100 µg/mL of streptomycin, 100 units/mL of penicillin, 10 ng/mL of rhIL-15, and 500 ill of rhIL-2. Culture of all cells was performed in a 5% CO₂ (95% air) environment at 37°C. For expression of the mesothelin-specific CAR, 5 × 10⁵ PBNK cells and the produced antigen receptor construct virus with an MOI of 2 were added to a 24-well plate, and then for induction of transduction, PGE2 (prostaglandin E2) and LentiBOOST (lentiviral gene transfer inducer) were used, thereby generating NK cells expressing the mesothelin-specific CAR. In order to analyze the phenotype of the generated cells expressing the mesothelin-specific CAR, the cells were rinsed twice with FACS buffer, and the rinsed cells were subjected to FACS analysis using Pacific blue-conjugated anti-CD3 antibody, APC-conjugated anti-CD56 antibody, Bv785-conjugated anti-CD16 antibody, and PE-conjugated MSLN antigen. However, in the drawings and tables, mock represents the results for NK cells transfected with an empty vector.

**[Table 22]**

| | Mock | #19-1 |
|---|---|---|
| NK+ (CD3⁻,CD56⁺) | 81.82% | 83.64% |
| Activity (CD16⁺) | 85.89% | 71.65% |

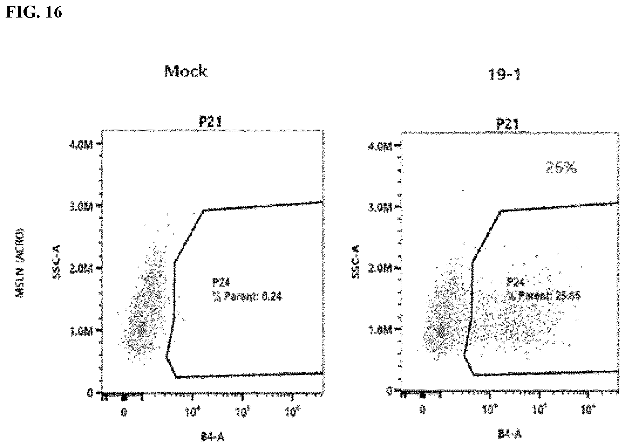

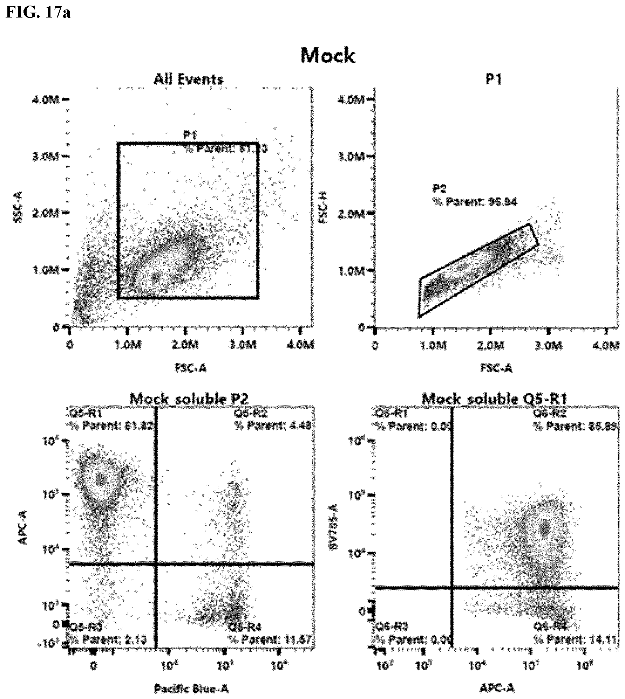

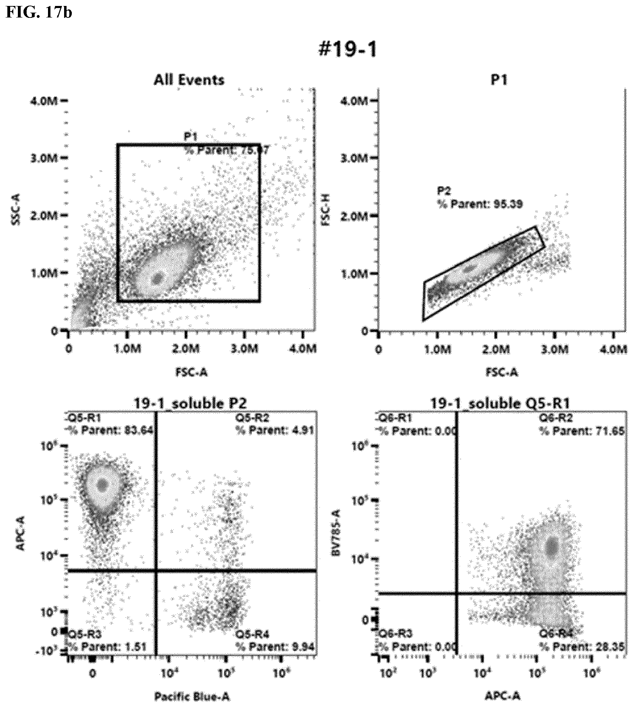

As a result, as shown in FIGS. 16 and 17 and Table 22 , it could be confirmed that the cells induced through the above-described process were CD3⁻CD56⁺ NK cells, and CD16+, an NK cell activity marker, was expressed in 71.65% of the cells, and the mesothelin-specific CAR was expressed in 25% or more of the cells.

### [Example 101 Evaluation of Cancer Cell Killing Ability of NK Cells Expressing Mesothelin-Specific Chimeric Antigen Receptor (CAR)

In order to confirm the cytotoxicity of the mesothelin-specific CAR-expressing NK cells generated in Example 9 above against cancer cells, the mesothelin-specific CAR-expressing NK cells were co-cultured with Capan-2 pancreatic cancer cells, HepG2 liver cancer cells, and MDA-MB-231 breast cancer cells, known to express mesothelin at high levels. After 4 hours of co-culture, dead or damaged cells were stained with PI (propidium iodide). Thereafter, the killing ability of the CAR-expressing NK cells against the cancer cells was analyzed by FACS.

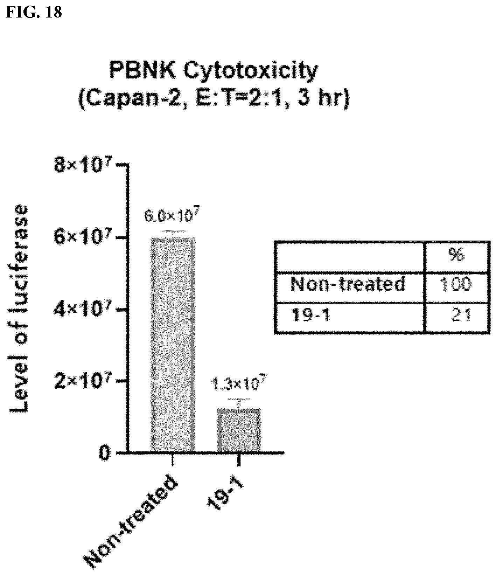

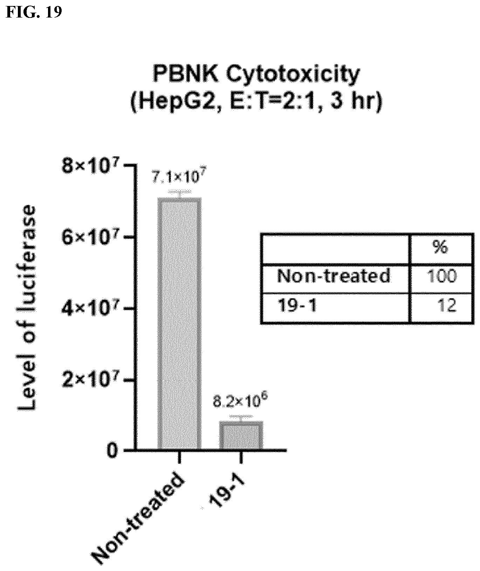

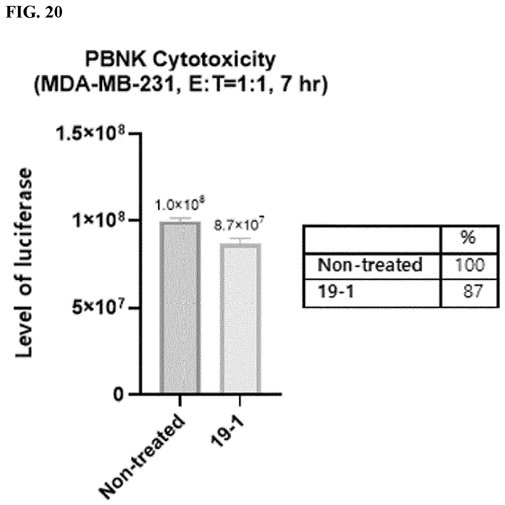

As shown in FIGS. 18 to 20, it could be confirmed that the mesothelin-specific CAR-expressing NK cells according to the present invention had excellent killing ability against all of the mesothelin-expressing pancreatic cancer cells, liver cancer cells, and breast cancer cells.

The above description of the present invention is exemplary, and those of ordinary skill in the art will appreciate that the present invention can be easily modified into other specific forms without departing from the technical spirit or essential characteristics of the present invention. Therefore, it should be understood that the embodiments described above are illustrative in all respects and not restrictive.

### Industrial Applicability

The present invention relates to antibodies or antigen-binding fragments capable of binding specifically to mesothelin antigen and various uses thereof.

## Claims

1. An antibody or antigen-binding fragment which binds specifically to mesothelin (MSLN) and comprises:
1) a heavy chain variable region comprising a heavy chain CDR1 comprising the amino acid sequence set forth in SEQ ID NO: 29; a heavy chain CDR2 comprising the amino acid sequence set forth in SEQ ID NO: 5; a heavy chain CDR3 comprising the amino acid sequence set forth in SEQ ID NO: 32; a light chain CDR1 comprising the amino acid sequence set forth in SEQ ID NO: 10; a light chain CDR2 comprising the amino acid sequence set forth in SEQ ID NO: 12; and a light chain CDR3 comprising the amino acid sequence set forth in SEQ ID NO: 14;
2) a heavy chain variable region comprising a heavy chain CDR1 comprising the amino acid sequence set forth in SEQ ID NO: 3, a heavy chain CDR2 comprising the amino acid sequence set forth in SEQ ID NO: 5, and a heavy chain CDR3 comprising the amino acid sequence set forth in SEQ ID NO: 7; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 14; or
3) a heavy chain variable region comprising a heavy chain CDR1 comprising the amino acid sequence set forth in SEQ ID NO: 17, a heavy chain CDR2 comprising the amino acid sequence set forth in SEQ ID NO: 19, and a heavy chain CDR3 comprising the amino acid sequence set forth in SEQ ID NO: 21; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 26.

2. The antibody or antigen-binding fragment of claim 1, wherein the antigen-binding fragment comprises a single-chain antibody, Fd, Fab, Fab', F(ab')2, dsFv, or scFv.

3. The antibody or antigen-binding fragment of claim 1, wherein the antibody comprises a polyclonal antibody, a monoclonal antibody, a whole antibody, a chimeric antibody, a human antibody, a humanized antibody, a bivalent bispecific molecule, a minibody, a domain antibody, a bispecific or multispecific antibody, an immune cell-engaging bispecific or multispecific antibody, an antibody mimetic, a unibody, a diabody, a triabody, or a tetrabody.

4. The antibody or antigen-binding fragment of claim 1, which further comprises:
a heavy chain FR1 comprising the amino acid sequence of at least one of SEQ ID NOs: 2 or 16; a heavy chain FR2 comprising the amino acid sequence of at least one of SEQ ID NOs: 4 or 18; a heavy chain FR3 comprising the amino acid sequence of at least one of SEQ ID NOs: 6 or 20; and a heavy chain FR4 comprising the amino acid sequence of at least one of SEQ ID NOs: 8 or 22, and
a light chain FR1 comprising the amino acid sequence of at least one of SEQ ID NOs: 9 or 23; a light chain FR2 comprising the amino acid sequence of at least one of SEQ ID NOs: 11 or 24; a light chain FR3 comprising the amino acid sequence of at least one of SEQ ID NOs: 13 or 25; and a light chain FR4 comprising the amino acid sequence of at least one of SEQ ID NOs: 15 or 27.

5. The antibody or antigen-binding fragment of claim 1, which further comprises:
a heavy chain FR1 comprising the amino acid sequence set forth in SEQ ID NO: 28; a heavy chain FR2 comprising the amino acid sequence set forth in SEQ ID NO: 30; a heavy chain FR3 comprising the amino acid sequence set forth in SEQ ID NO: 31; a heavy chain FR4 comprising the amino acid sequence set forth in SEQ ID NO: 33; a light chain FR1 comprising the amino acid sequence set forth in SEQ ID NO: 34; a light chain FR2 comprising the amino acid sequence set forth in SEQ ID NO: 35; a light chain FR3 comprising the amino acid sequence set forth in SEQ ID NO: 36; and a light chain FR4 comprising the amino acid sequence set forth in SEQ ID NO: 37.

6. The antibody or antigen-binding fragment of claim 1, which comprise at least one of the following antibodies or antigen-binding fragments:
1) a heavy chain variable region comprising the amino acid sequence set forth in SEQ ID NO: 46; and a light chain variable region comprising the amino acid sequence set forth in SEQ ID NO: 47;
2) an antibody or antigen-binding fragment comprising a heavy chain variable region comprising the amino acid sequence set forth in SEQ ID NO: 38, and a light chain variable region comprising the amino acid sequence set forth in SEQ ID NO: 39; and
3) an antibody or antigen-binding fragment comprising a heavy chain variable region comprising the amino acid sequence set forth in SEQ ID NO: 42, and a light chain variable region comprising the amino acid sequence set forth in SEQ ID NO: 43.

7. A nucleic acid molecule encoding the antibody or antigen-binding fragment of any one of claims 1 to 6.

8. A recombinant expression vector comprising the nucleic acid molecule of claim 7.

9. A host cell transfected with the expression vector of claim 8.

10. An immunoconjugate comprising the antibody or antigen-binding fragment of any one of claims 1 to 6 and a cytotoxic agent.

11. A composition for use in a method of diagnosing mesothelin-related disease, comprising the antibody or antigen-binding fragment of any one of claims 1 to 6 as an active ingredient.

12. A chimeric antigen receptor (CAR) comprising an antigen-binding domain which binds specifically to mesothelin (MSLN), the chimeric antigen receptor comprising an antigen-binding fragment comprising:
1) a heavy chain CDR1 comprising the amino acid sequence set forth in SEQ ID NO: 29; a heavy chain CDR2 comprising the amino acid sequence set forth in SEQ ID NO: 5; a heavy chain CDR3 comprising the amino acid sequence set forth in SEQ ID NO: 32; a light chain CDR1 comprising the amino acid sequence set forth in SEQ ID NO: 10; a light chain CDR2 comprising the amino acid sequence set forth in SEQ ID NO: 12; and a light chain CDR3 comprising the amino acid sequence set forth in SEQ ID NO: 14;
2) a heavy chain variable region comprising a heavy chain CDR1 comprising the amino acid sequence set forth in SEQ ID NO: 3, a heavy chain CDR2 comprising the amino acid sequence set forth in SEQ ID NO: 5, and a heavy chain CDR3 comprising the amino acid sequence set forth in SEQ ID NO: 7; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 14; or
3) a heavy chain variable region comprising a heavy chain CDR1 comprising the amino acid sequence set forth in SEQ ID NO: 17, a heavy chain CDR2 comprising the amino acid sequence set forth in SEQ ID NO: 19, and a heavy chain CDR3 comprising the amino acid sequence set forth in SEQ ID NO: 21; and a light chain variable region comprising a light chain CDR1 comprising the amino acid sequence set forth in SEQ ID NO: 10, a light chain CDR2 comprising the amino acid sequence set forth in SEQ ID NO: 12, and a light chain CDR3 comprising the amino acid sequence set forth in SEQ ID NO: 26.

13. The chimeric antigen receptor (CAR) of claim 12, wherein the chimeric antigen receptor comprises
1) an antigen-binding fragment comprising a heavy chain variable region comprising the amino acid sequence set forth in SEQ ID NO: 46; and a light chain variable region comprising the amino acid sequence set forth in SEQ ID NO: 47;
2) an antigen-binding fragment comprising a heavy chain variable region comprising the amino acid sequence set forth in SEQ ID NO: 38, and a light chain variable region comprising the amino acid sequence set forth in SEQ ID NO: 39; or
3) an antigen-binding fragment comprising a heavy chain variable region comprising the amino acid sequence set forth in SEQ ID NO: 42, and a light chain variable region comprising the amino acid sequence set forth in SEQ ID NO: 43.

14. An immune effector cell expressing the chimeric antigen receptor of claim 12 or 13.

15. A pharmaceutical composition for use in a method of preventing or treating mesothelin-related disease, comprising the antibody or antigen-binding fragment of any one of claims 1 to 6 or the immune effector cell of claim 14 as an active ingredient.
